(19)

Europäisches
Patentamt
European
Patent Office
Office européen
des brevets

(11) **EP 3 310 925 B1**

(12) **EUROPEAN PATENT SPECIFICATION**

(45) Date of publication and mention
of the grant of the patent:
**16.09.2020 Bulletin 2020/38**

(21) Application number: **16731303.0**

(22) Date of filing: **17.06.2016**

(51) Int Cl.:
***G16Z 99/00*** *(2019.01)*

(86) International application number:
**PCT/GB2016/051829**

(87) International publication number:
**WO 2016/203266 (22.12.2016 Gazette 2016/51)**

(54) **METHOD, APPARATUS AND ELECTROCHEMICAL TEST DEVICE**

VERFAHREN, VORRICHTUNG UND ELEKTROCHEMISCHE VORRICHTUNG

PROCÉDÉ, APPAREIL ET DISPOSITIF DE TEST ÉLECTROCHIMIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.06.2015 GB 201510765**

(43) Date of publication of application:
**25.04.2018 Bulletin 2018/17**

(73) Proprietor: **Inside Biometrics International Limited
Dingwall, Scotland IV15 9QF (GB)**

(72) Inventors:
• **SAINI, Selwayan
Dingwall
Inverness IV15 9QF (GB)**
• **RODGERS, James
Dingwall
Inverness IV15 9QF (GB)**
• **BASKEYFIELD, Damian
Dingwall
Inverness IV15 9QF (GB)**

(74) Representative: **Iqbal, Md Mash-Hud
Marks & Clerk LLP
62-68 Hills Road
Cambridge CB2 1LA (GB)**

(56) References cited:
**EP-A1- 2 308 991     WO-A1-2009/060432
WO-A1-2013/036617     WO-A1-2016/097768**

WO-A1-2016/174454     WO-A1-2016/174460
US-A1- 2003 068 666     US-A1- 2014 124 384

• **CHRISTINA VOULGARI ET AL: "The Performance
of a Glucose-Ketone Meter in the Diagnosis of
Diabetic Ketoacidosis in Patients with Type 2
Diabetes in the Emergency Room", DIABETIS
TECHNOLOGY & THERAPEUTICS, vol. 12, no. 7,
1 July 2010 (2010-07-01), pages 529-535,
XP055297000, MARY ANN LIEBERT,
LARCHMONT, NY; US ISSN: 1520-9156, DOI:
10.1089/dia.2010.0011**
• **S. MISRA ET AL: "Utility of ketone measurement
in the prevention, diagnosis and management of
diabetic ketoacidosis", DIABETIC MEDICINE.,
vol. 32, no. 1, 19 January 2015 (2015-01-19), pages
14-23, XP055296993, GB ISSN: 0742-3071, DOI:
10.1111/dme.12604**
• **Xinxia Cai ET AL: "The Development of
Biosensors and Biochips in IECAS", Lecture
Notes in Computer Science (including subseries
Lecture Notes in Artificial Intelligence and
Lecture Notes in Bioinformatics), 1 January 2004
(2004-01-01), pages 552-525, XP055065242,
Retrieved from the Internet:
URL:http://rd.springer.com/content/pdf/10.
1007/978-3-540-30141-7_76.pdf [retrieved on
2013-06-04]**
• **SHAO-PENG CHEN ET AL: "Multi-parameter
detection of diabetes mellitus on multichannel
poly(dimethylsiloxane) analytical chips coupled
with nanoband microelectrode arrays",
ELECTROPHORESIS, vol. 31, no. 18, 19 August
2010 (2010-08-19), pages 3097-3106,
XP055316177, DE ISSN: 0173-0835, DOI:
10.1002/elps.201000181**

EP 3 310 925 B1

## Description

### Technical Field

The present disclosure relates to test devices, apparatuses and methods for detecting one or more analytes in a fluid sample. In particular, the present disclosure relates to methods and apparatuses suitable for processing data from a test device such as an electrochemical test strip. The present disclosure further relates to electrochemical test devices for determining concentrations of one or more analytes in a fluid sample.

### Background

The detection and measurement of substances, chemicals, or analytes in a bodily fluid sample is useful in a variety of applications, such as in fitness monitors or in the medical device industry. For example, an individual may choose to monitor a concentration of an analyte such as lactate in his or her bloodstream in order to determine whether or not a chosen fitness regime is effective.

As another example, people with diabetes need to regularly monitor the concentration of glucose in their bloodstream in order to determine if they are in need of glucose or insulin or other diabetes medication. Diagnostic devices and kits have been developed over the years to allow a diabetic individual to autonomously determine the concentration of glucose in their bloodstream, in order to better anticipate the onset of hyperglycaemia or hypoglycaemia and take any necessary action. These devices and kits are often referred to as Self-Monitoring of Blood Glucose (SMBG) devices and kits.

Another of the primary reasons why people with diabetes perform SMBG tests is to identify situations in which they may be at risk of ketoacidosis which is usually associated with very high blood glucose concentrations.

A severe insulin insufficiency (hypoinsulinaemia) is almost always also associated with sustained, highly elevated blood glucose concentration. Such hypoinsulinaemia can lead to the adverse condition diabetic ketoacidosis (DKA). Failure to detect DKA in a timely fashion or to appropriately manage this complication whenever it arises can result in a very serious medical emergency. Conversely if caught early enough and managed appropriately (e.g. with rehydration and therapeutic insulin), adverse outcomes can be fully mitigated, with medical emergencies prevented. Preventable hospitalisations and deaths can thus be avoided with a combination of relatively straightforward prophylactic measures and/or benign therapeutic interventions.

DKA is usually associated with blood glucose concentrations of greater than 300mg/dl. Instances of DKA where the blood glucose concentration is less than 200mg/dl have been relatively unusual.

Conventional approaches to DKA detection and prevention within non-clinical settings are based on first detecting high blood glucose by using SMBG measurement. Only when blood glucose is elevated, is the potential onset of DKA checked through subsequent measurement of blood or urinary ketones.

Therefore where ketoacidosis is associated with elevated blood glucose or where nausea, vomiting or abdominal pain is present, the subsequent presence of ketones and attendant risk of DKA should in theory be picked up by any user who diligently follows existing advice.

However, aside the fact that users may not always be diligent in following the recommendations as laid down, there remains a small but significant number of instances where ketoacidosis occurs in the absence of elevated blood glucose. This euglycaemic ketoacidosis can arise from metabolic changes resulting from, for example, carbohydrate restriction (which may result from starvation (e.g. associated with anorexia)), or diet (e.g. carbohydrate restricted diets), emesis (e.g. due to illness), or metabolic changes resulting from alcohol use or dependency, or metabolic changes occurring in pregnancy, or metabolic changes resulting from certain prescription drug use. In these instances a user could still miss a DKA event onset even where diligently following existing best advice.

Since it is the hypoinsulinaemia that stimulates the ketogenic pathways that ultimately results in DKA, irrespective of the actual blood glucose level, then any circumstances in which an insulin insufficiency coincides with low carbohydrate availability has the potential to result in a euglycaemic ketoacidosis event that could, with the currently existing standard of care, be susceptible to misdiagnosis for example, with the attendant increased risks of an adverse outcome for the sufferer.

Furthermore, some drugs including some diabetes management drugs may have the potential to increase the likelihood of DKA, and may also increase the likelihood that any such DKA is associated with euglycaemia.

For example, the SGLT2 inhibitor class of drugs (for example Dapagliflozon, Canagliflozin and Empagliflozin) have recently been licensed for use in diabetes management. These drugs work by increasing the amount of glucose from blood which is excreted through the kidneys. Their introduction has been associated with DKA as a possible side effect and several instances of SGLT2-associated DKA episodes have been associated with euglycaemia. As these highly effective drugs (and others with similar mode of action response profiles) become more widely adopted, there exists a concomitant danger of ever increasing occurrences of dangerous euglycaemic DKA episodes going undetected and untreated, with adverse outcomes for the patient.

Additionally, certain medications, including Biguanide drugs such as Metformin, Phenformin and Buformin (and including Metformin and SGLT2 inhibitor metformin combination therapies) have been linked with the occurrence of lactic acidosis. For example, a condition which has come to be designated as "MALA" (Metformin Associated Lactic Acidosis) has been identified. This exists within persons prescribed metformin based medications. Although rare, MALA is a life threatening complication, and if early detection and rapid treatment with haemodialysis is not effected, a mortality rate of up to 50% is known.

When trying to ascertain a level of an analyte in, for example, a blood sample, an individual will typically perform a finger stick using a lancing device to extract a small drop of blood from a finger or alternative site. An electrochemical test device, which is often a test strip, is then inserted into a diagnostic meter, and the sample is applied to the test strip. Through capillary action, the sample flows through a capillary channel across a measurement chamber of the device and into contact with one or more electrodes or conductive elements coated with sensing chemistry for interacting with a particular analyte or other specific chemical in the blood sample. The magnitude of the reaction is dependent on the concentration of the analyte in the blood sample. The diagnostic meter may detect the current generated by the reaction of the sensing chemistry with the analyte, and the result can be displayed to the individual.

Typically, such electrochemical test devices have a set of electrodes such as a counter/reference electrode and one or more working electrodes. Sensing chemistry is used which is typically tailored to the particular analyte or biometric of interest. An enzymatic electrode is a combination of an enzyme and an electrochemical transducer. The direct transfer of electrons between the enzyme and the electrode is generally not easy to achieve and so an electron transfer agent (or mediator) is sometimes used to transfer electrons between the enzyme and the electrode to facilitate the electrocatalysis. For example, when measuring the concentration of glucose in a sample, a glucose oxidase or a glucose dehydrogenase enzyme can be used in conjunction with a mediator such as potassium ferricyanide. When detecting other analytes, different enzymes may be used, such as $\beta$-hydroxybutyrate dehydrogenase for measuring the ketone body $\beta$-hydroxybutyrate or lactate oxidase for measuring lactate.

Conventionally, where highly elevated blood glucose readings occur within an SMBG measurement, users are often alerted to perform an additional check for the presence of ketone bodies either in their blood or urine. This enables the user to either ensure that DKA is not occurring, or to instigate appropriate and timely medical intervention in the event that DKA is occurring. At present, this necessitates the use of an additional separate test strip with an appropriate reagent chemistry which is distinct from the SMBG test strip itself. At present, users do not routinely screen for any risk of lactic acidosis in response to SMBG testing.

Whether ketone testing is done by urinalysis strips or by additional blood testing, conventionally, additional user steps, cost and inconvenience are involved for the user. Furthermore, some users may elect not to bother with the additional testing, and may miss the potentially dangerous onset of DKA as a result, and fail to treat appropriately, with the potential for adverse consequences.

## Summary

The invention is set out in the independent claim.

Thus we describe a method of processing data from a multi-analyte electrochemical test device, the method comprising:

determining a concentration of a first analyte in a fluid sample using first sensing chemistry, a first working electrode, and a counter electrode, wherein the first analyte is an acidosis-related analyte and comprises a ketone body;
determining a concentration of a second analyte in the fluid sample using second sensing chemistry, a second working electrode, and the counter electrode, wherein the second analyte is glucose;
displaying a first indication related to the determined concentration of the second analyte;
if the determined concentration of the first analyte is equal to or greater than a first threshold, wherein the first threshold is a user definable threshold, providing a user-selectable option to display a second indication related to the determined concentration of the first analyte.

Other preferred examples are defined in the dependent claims.

An acidosis-related analyte may be any analyte which may be associated with a reduction in pH or an increase in the anion gap within the blood, serum, plasma and other body tissues. The anion gap is the difference between the measured cations (positively charged electrolytes) and measured anions (negatively charged electrolytes) within the sample, usually expressed in milliequivalents per litre (mEq/L).

By providing a method as described, high levels of acidosis-related analytes such as lactate or ketones may be detected and conveyed to a user, even when glucose levels in the fluid sample are not indicative of hyperglycaemia, for example when the glucose levels in the fluid are at a normal, euglycaemic, level. Accordingly, a user may be alerted to conditions such as euglycaemic DKA, which is where ketoacidosis occurs as a result of hypoinsulinaemia, even in the absence of hyperglycaemia, for example because of an insufficiency of carbohydrate intake.

Examples described herein address the risk of DKA and enable effective early detection of DKA onset and prevention of DKA complications and hospitalisations, even where such DKA is associated with euglycaemic blood glucose readings. Other examples described herein address this risk and enable effective early detection of lactic acidosis onset and prevention of lactic acidosis associated complications and hospitalisations.

The risk of false alerts caused by spurious readings is minimised by utilisation of additional information pertaining to underlying risk factors, both acute and sustained, specific to a particular user.

A "Companion Health Management Tool" is disclosed which assists users, especially (although not exclusively) diabetic users, and their Health Care Professionals (HCPs) in managing their medications in such a way as to also optimise the benefit they receive from their condition-controlling medications while minimising the concomitant risk of potentially dangerous complications such as acidosis, over the medium to long term.

Additionally, there are disclosed devices and methods which facilitate early and easy disambiguation between distinct types of acidosis which may differ in their severity, optimum treatment protocols, and prognoses, such as lactic acidosis, diabetic ketoacidosis and euglycaemic ketoacidosis, and to thereby ensure that the most appropriate medical intervention can be delivered in a timely manner. For example severe lactic acidosis can warrant corrective haemodialysis treatment sometimes necessitating rapid hospitalisation, whereas mild DKA can often be self-managed or managed in a primary care setting with insulin and oral rehydration therapy. Euglycaemic ketoacidosis may require carbohydrate intake as well as insulin therapy, whereas in hyperglycaemic DKA, carbohydrate intake is specifically contra-indicated. Furthermore, where the underlying cause of DKA is potentially related to a specific medications, changes in dose or dosing frequency may be warranted, and disambiguation of the specific type of acidosis can be useful in establishing which medication needs to be adjusted. For example lactic acidosis in the absence of ketones may indicate that Biguanide medication might be the more likely implicated, whereas euglycaemic DKA might imply that SGLT2 medication is potentially the more likely implicated medication.

There is disclosed a blood ketone measurement which is effected simultaneously with the initial blood glucose test, optionally from the same sample, further optionally from within the same strip, with the user being advised automatically about the potential onset of DKA, in the event that an elevated blood ketone concentration had been detected, so that early detection and appropriate management and mitigation could be effected in the least deleterious manner.

There is disclosed a blood lactate measurement which is effected simultaneously with the initial blood glucose test, optionally from the same sample, further optionally from within the same strip, with the user being advised automatically about the potential onset of lactic acidosis, in the event that an elevated blood lactate concentration had been detected, so that early detection and appropriate management and mitigation could be effected in the least deleterious manner.

There is further disclosed a blood lactate measurement and a blood ketone measurement which are effected substantially simultaneously with the initial blood glucose test, optionally from the same sample, further optionally from within the same strip, with the user being advised automatically about the potential onset of any acidosis, with a concomitant disambiguation of the type of acidosis risk, in the event that an elevated blood lactate concentration or blood ketone concentration, or both had been detected, so that early detection and appropriate management and mitigation could be effected in the least deleterious manner.

There is further disclosed tracking and trending of measurements using such systems over a prolonged duration in conjunction with individualised information pertaining to specific acidosis risk factors and medication types and dosages, in such a way as to facilitate optimisation of treatment for the maximum benefit with respect to the underlying condition being treated, along with the simultaneous minimisation of acidosis episodes and other risk complications (e.g. hypoglycaemia).

Examples include a method and apparatus for the detection, prevention and management of ketoacidosis risks, including types of euglycaemic ketoacidosis, and ketoacidosis associated with medication.

Examples include a method and apparatus for the detection, prevention and management of lactic acidosis risks, including lactic acidosis associated with medication, for example diabetes medications which contain biguanide drugs such as metformin, phenformin or buformin.

Further examples include a method and apparatus for the substantially simultaneous detection, prevention and management of both ketoacidosis and lactic acidosis, with simultaneous disambiguation of the subtype of acidosis risk likely to be present.

Euglycaemic DKA is a fairly rare occurrence. There is therefore a need to distinguish between occasional spurious results that will inevitably occur, and genuine medical emergencies which need to be dealt with as a matter of urgency. Examples encompass apparatuses and systems which have been configured with appropriate methods to make such distinctions more easily possible.

Furthermore, because lactic acidosis is a fairly rare occurrence, there is a need to distinguish between occasional spurious results that will inevitably occur, and genuine medical emergencies which need to be dealt with as matter of urgency. Examples encompass apparatuses and systems which have been configured with appropriate methods to make such distinctions more easily possible.

Additionally, the apparatuses and systems disclosed herein can also be configured to include appropriate methods which enable medication (e.g. insulin, SGLT2 inhibitors, biguanides such as metformin and SGLT2/metformin combinations) to be titrated and dose adjusted and/or dose and time adjusted appropriately to ensure that adverse DKA events or lactic acidosis events can be avoided in the first instance, with treatment regimens thereby being optimised and individualised according to an individual's risk profile and user experience.

Examples include a meter and test strip for periodic simultaneous determination of blood glucose and blood β-hydroxybutyrate concentration. Other examples include a meter and test strip for the simultaneous determination of both blood glucose and blood lactate concentration. Further examples include a meter and test strip for the substantially simultaneous determination of blood lactate, blood β-hydroxybutyrate and blood glucose.

The present disclosure further provides a Companion Health Management tool within the test system that controls the communication of the blood glucose readings and/or blood ketone readings and/or blood lactate readings to the user and advises the user appropriately, based upon a combination of the individual measurements obtained and at least some the following elements:

(i) patient related information entered into the meter upon system set up by a user or health care professional (HCP), relevant to the risk of DKA or lactic acidosis;
(ii) prior meter readings;
(iii) user inputs in response to prompts;
(iv) event flags;
(v) trending information and/or averages captured by the meter across a series of measurements; and
(vi) configurable thresholds (CTs) set by the user and/or their HCP which enable alerting to be set according to individualised circumstances, medication types, dosages, and risk profiles.

The configurable thresholds of embodiments that control the alerting features are arranged to be modified and individualised in such a way that medication can thereby be optimally titrated so as to achieve the optimum balance of risk and benefit for a specific user according to their actual individual responses and measurements, (most likely, working in partnership with their HCP).

It is known that because of an individual's underlying metabolic health, risk factors, medication types and response history, the same measurement result across different individuals may have different underlying aetiologies and may necessitate very different courses of action. Readings which could signify the onset of a serious problem in one individual prompting a timely alert and appropriate medical intervention that could otherwise have been missed, could signify no problem in another with a different aetiology and any such alert would carry a nuisance value. The use of individually configurable thresholds for lactate and ketones alerts, in accordance with the present disclosure, allows users to tailor the system to their own metabolic profile and thereby maximise the value of any alerts while minimising the nuisance value of false alarms

The present disclosure describes improved methods of operating an apparatus or test meter for use with a multi-analyte electrochemical test device in order to monitor glucose and ketone levels. The present disclosure further describes a companion health management tool.

Further optional features will be appreciated from the following description.

**Brief Description of the Drawings**

Illustrative embodiments of the present disclosure will now be described, by way of example only, with reference to the drawings. In the drawings:

Figure 1 shows an exploded view of an electrochemical test device;
Figure 2 shows a plan view of some layers of an electrochemical test device;
Figure 3 illustrates bio-electrocatalysis at an electrode;
Figure 4 shows reagent layers that may be applied to two working electrodes;
Figure 5 shows reagent layers that may be applied to two working electrodes;
Figure 6 shows an apparatus for use with an electrochemical test device;
Figure 7 is a flowchart of a method;
Figure 8 is a flowchart of a method;
Figure 9 is a high level flow diagram;
Figures 10a-10c show screenshots displayed on an apparatus or test meter;
Figures 11a-11c show screenshots displayed on an apparatus or test meter;

Figure 12 shows a screenshot displayed on an apparatus or test meter;
Figure 13 is a high level flow diagram;
Figure 14 is a high level flow diagram;
Figure 15 shows an exploded view of an electrochemical test device;
Figure 16 shows a plan view of some layers of an electrochemical test device; and
Figures 17a-17e show screenshots displayed on an apparatus or test meter.

Throughout the description and the drawings, like reference numerals refer to like parts.

**Description**

Whilst various embodiments of the invention are described below, the invention is not limited to these embodiments, and variations of these embodiments may be made without departing from the scope of the invention.

We describe in further detail herein a method comprising determining a concentration of a first analyte in a fluid sample using first sensing chemistry. The first analyte may be an acidosis-related analyte. The method may further comprise determining a concentration of a second analyte in the fluid sample using second sensing chemistry. The second analyte may be glucose. The method may further comprise displaying a first indication related to the determined concentration of the second analyte. The method may further comprise, if the determined concentration of the first analyte is equal to or greater than a first threshold, providing a user-selectable option to display a second indication related to the determined concentration of the first analyte.

The following disclosure further describes an apparatus for processing data from a test device. The apparatus may comprise a memory storing instructions to perform a method as disclosed herein. The apparatus may further comprise a processor which may be configured to perform the instructions stored in the memory. The apparatus may comprise circuitry for receiving an output signal generated from a fluid sample.

The following disclosure further describes a machine-readable medium. The machine-readable medium may have instructions stored thereon. The instructions may be configured such that when read by a machine, the instructions cause a method as disclosed herein to be carried out.

The following disclosure further describes a system. The system may comprise an apparatus as described herein for processing data from a test device. The system may further comprise a test device comprising a first working electrode having first sensing chemistry for detecting the first analyte; and a second working electrode having second sensing chemistry for detecting the second analyte.

The following disclosure further describes an electrochemical test device for determining concentrations of multiple analytes in a fluid sample. The electrochemical test device may comprise a first working electrode for first sensing chemistry for detecting a first analyte. The electrochemical test device may further comprise a second working electrode for second sensing chemistry for detecting a second analyte. The electrochemical test device may further comprise a third working electrode for third sensing chemistry for detecting a third analyte.

Throughout this specification, reference is made to directional terms such as "above" and "below", or "upper" and "lower". References made to such terms are purely indicative of relative positions of the features of examples disclosed herein. For example, wherever there is mention of a cover above a spacer layer and an insulator layer below the spacer layer, it should be understood that the cover and the insulator layer are formed on opposite sides of the spacer layer. That is, directional terms such as those described herein do not refer to a direction relative to a viewpoint of an observer, but instead should be considered in all aspects as relative terms.

Various additional details of aspects of electrochemical test devices are described in the following commonly assigned patent applications (denoted collectively herein as the "related applications"). These related applications include the EP patent application No. 16720526.9 entitled "Electrochemical test device"; PCT publication No. WO/2016/174457 entitled "Electrochemical test device"; the EP patent application No. 16720528.5 entitled "Electrochemical test device"; the EP patent application No. 16720525.1 entitled "Electron transfer agent"; and the EP patent application No. 16720529.3 entitled "Electrochemical test device".

The term "alkyl", used alone or as part of a larger moiety, refers to a straight or branched chain aliphatic group having from 1 to 12 carbon atoms. The alkyl group therefore has 1, 2, 3, 4, 5, 6, 7, 8, 9, 10, 11 or 12 carbon atoms. For purposes of the present disclosure, the term "alkyl" will be used when the carbon atom attaching the aliphatic group to the rest of the molecule is a saturated carbon atom. However, an alkyl group may include unsaturation at other carbon atoms. Thus, alkyl groups include, without limitation, methyl, ethyl, propyl, allyl, propargyl, butyl, pentyl, and hexyl.

The term "amine" may refer to a primary, secondary or tertiary amine. The amine will generally be NRR'R", where R, R' and R" are each selected from hydrogen or alkyl. Any suitable alkyl group may be used. Preferred alkyl group will be $C_1$, $C_2$, $C_3$, $C_4$, $C_5$, $C_6$. Preferably, an amine is $NH_3$.

The term "heteroaryl" means a monocyclic or bicyclic radical of 5 to 12 ring atoms having at least one aromatic ring containing one, two, or three ring heteroatoms selected from N, O or S, the remaining ring atoms being C. The attachment point of the heteroaryl radical may be via the heteroatom. The heteroaryl rings may be optionally substituted as defined herein. Examples of heteroaryl moieties include, but are not limited to, optionally substituted imidazolyl, oxazolyl, isoxazolyl, thiazolyl, isothiazolyl, oxadiazolyl, thiadiazolyl, pyrazinyl, thienyl, benzothienyl, thiophenyl, furanyl, pyranyl, pyridyl, pyrrolyl, pyrazolyl, pyrimidyl, quinolinyl, isoquinolinyl, benzofuryl, benzothiophenyl, benzothiopyranyl, benzimidazolyl, benzooxazolyl, benzooxadiazolyl, benzothiazolyl, benzothiadiazolyl, benzopyranyl, indolyl, isoindolyl, triazolyl, triazinyl, quinoxalinyl, purinyl, quinazolinyl, quinolizinyl, naphthyridinyl, pteridinyl, carbazolyl, azepinyl, diazepinyl, acridinyl and the like, including partially hydrogenated derivatives thereof.

The term "halide" refers to a substituent which is fluoro, chloro, bromo or iodo.

The term "substituted", as used herein, means that a hydrogen radical of the designated moiety is replaced with the radical of a specified substituent, provided that the substitution results in a stable or chemically feasible compound. The phrase "one or more substituents", as used herein, refers to a number of substituents that equals from one to the maximum number of substituents possible based on the number of available bonding sites, provided that the above conditions of stability and chemical feasibility are met. Unless otherwise indicated, an optionally substituted group may have a substituent at each substitutable position of the group, and the substituents may be either the same or different.

An electron transfer agent (or redox mediator) is an agent for transferring electrons between an analyte or an analyte-reduced or analyte-oxidized enzyme and an electrode, either directly, or via one or more additional electron transfer agents.

An electron transfer agent as disclosed herein may be distinguishable by its standard redox potential i.e. a standard hydrogen electrode (SHE) at Standard Temperature and Pressure (25°C and 1 atm).

A method of processing data from a test device is disclosed. The method comprises determining a concentration of a first analyte in a fluid sample using first sensing chemistry, wherein the first analyte is an acidosis-related analyte. The method further comprises determining a concentration of a second analyte in the fluid sample using second sensing chemistry, wherein the second analyte is glucose. The method further comprises displaying a first indication related to the determined concentration of the second analyte. The method further comprises, if the determined concentration of the first analyte is equal to or greater than a first threshold, providing a user-selectable option to display a second indication related to the determined concentration of the first analyte.

The test device may be an electrochemical test device. The electrochemical test device may be a multi-analyte electrochemical test device.

The step of determining a concentration of a second analyte in the fluid sample may comprise determining that the concentration of the second analyte is indicative of euglycaemia.

The step of determining a concentration of a second analyte in the fluid sample may comprise determining that the concentration of the second analyte is less than or equal to 33 mmol/L and greater than or equal to 4 mmol/L.

The step of determining a concentration of a second analyte in the fluid sample may comprise determining that the concentration of the second analyte is less than or equal to 11 mmol/L and greater than or equal to 4 mmol/L.

The step of determining a concentration of a second analyte in the fluid sample may comprise determining that the concentration of the second analyte is indicative of hypoglycaemia.

The step of determining a concentration of a second analyte in the fluid sample may comprise determining that the concentration of the second analyte is indicative of hyperglycaemia.

The method may further comprise receiving user selection of the user-selectable option and, in response to receiving user selection of the user-selectable option, stopping display of the first indication and displaying the second indication.

Determining a concentration of a first analyte in the fluid sample may comprise determining that the concentration of the first analyte is indicative of an acidosis-related condition. The acidosis-related condition is diabetic ketoacidosis, DKA. The acidosis-related condition is euglycaemic diabetic ketoacidosis. The acidosis-related condition is Metformin associated lactic acidosis, MALA.

The first threshold may be a user definable threshold.

The first analyte may comprise a ketone body. The first analyte may comprise β-hydroxybutyrate. The first threshold may be about 0.1 mmol/L. The first threshold may be about 0.6 mmol/L. The first threshold may be about 1.5 mmol/L.

The first analyte may comprise lactate. The first threshold may be about 2.5 mmol/L. The first threshold may be about 3.5 mmol/L. The first threshold may be about 5 mmol/L.

If the determined concentration of the first analyte is greater than or equal to a second threshold, the second threshold being greater than the first threshold, the second indication may comprise a warning concerning the determined concentration of the first analyte.

The second threshold may be a user definable threshold. The second threshold may be about 0.6 mmol/L. The second threshold may be about 1.5 mmol/L. The second threshold may be about 3 mmol/L. The second threshold may be about 5 mmol/L. The second threshold may be about 4 mmol/L. The second threshold may be about 3.5 mmol/L.

The method may further comprise receiving input, wherein the input comprises at least a selection from the group comprising:

(i) whether the user is pregnant/ has gestational diabetes
(ii) whether the user is prescribed insulin, and optionally the dose taken as well as optionally the insulin sensitivity factor of the individual
(iii) whether the user is prescribed SGLT2 medication (and optionally the dose taken)
(iv) whether the user is prescribed metformin or SGLT2 inhibitor metformin combination therapy.
(v) whether the user suffers from alcohol dependency
(vi) whether the user suffers from an eating disorder such as anorexia or bulimia
(vii) whether the user is following a carbohydrate restricted diet
(viii) whether the user has previously experienced a diabetic ketoacidosis, DKA, episode in the absence of elevated blood glucose

The method may further comprise receiving input, wherein the input comprises information pertaining to at least one of the following categories:

(i) whether the user has been in a state of fast;
(ii) whether the user has consumed alcohol;
(iii) whether the user has suffered from nausea, vomiting, abdominal pain, shortness of breath, excessive urination, or mental confusion;
(iv) whether the user has undertaken anaerobic exercise.

The method may further comprise logging the determined concentration of the first analyte and the determined concentration of the second analyte. The method may further comprise providing an indication of a trend in the logged determined concentration of the first analyte and the logged determined concentration of the second analyte.
The method may further comprise logging whether a transient risk of an acidosis-related condition was absent, present or not assessed.
The method may further comprise providing an output to display, the output comprising at least one of a prompt to repeat the test or an indication of a suspected condition of a user.
The first indication may comprise a numerical value. The first indication may comprise an informative symbol. The second indication may comprise a numerical value. The second indication may comprise an informative symbol. The method may further comprise determining a concentration of a third analyte in the fluid sample using third sensing chemistry, wherein the third analyte is an acidosis-related analyte. The method may further comprise, if the determined concentration of the third analyte is equal to or greater than a third threshold, providing a user-selectable option to display a third indication related to the determined concentration of the third analyte.
The method may further comprise communicating with a third party, such as a health care provider, over a communications network.
An apparatus for processing data from a test device is provided. The apparatus comprises a memory storing instructions to perform a method as disclosed herein. The apparatus further comprises a processor configured to perform the instructions stored in the memory.
A machine-readable medium is provided having instructions stored thereon. The instructions are configured such that when read by a machine, the instructions cause a method as disclosed herein to be carried out.
A system is provided. The system comprises an apparatus for processing data from a test device, as disclosed herein. The system further comprises a test device comprising a first working electrode having first sensing chemistry for detecting the first analyte, and a second working electrode having second sensing chemistry for detecting the second analyte. The test device may be an electrochemical test device. The electrochemical test device may be a multi-analyte electrochemical test device. The test device may further comprise a third working electrode having third sensing chemistry for detecting a third analyte.
An electrochemical test device for determining concentrations of multiple analytes in a fluid sample is provided. The electrochemical test device comprises a first working electrode for first sensing chemistry for detecting a first analyte. The electrochemical test device further comprises a second working electrode for second sensing chemistry for detecting a second analyte. The electrochemical test device further comprises a third working electrode for third sensing chemistry for detecting a third analyte. The first working electrode may have sensing chemistry for detecting the first analyte. The second working electrode may have sensing chemistry for detecting the second analyte. The third working electrode may have sensing chemistry for detecting the third analyte. The first analyte may be an acidosis-related analyte. The first analyte may comprise a ketone body. The first analyte may comprise $\beta$-hydroxybutyrate. The second analyte may comprise glucose. The third analyte may be an acidosis-related analyte. The third analyte may comprise lactate.
Multiple configurable thresholds may be used. The table below shows example thresholds that may be used (units of concentration shown in mmol/L).

| Example | Analyte | CT1 | CT2 | (CT3) | (CT4) | |
|---|---|---|---|---|---|---|
| 1 | Ketone | 0.1 | 0.6 | 1.5 | 3 | mMol/L |
| 2 | Ketone | 0.1 | 1.5 | N/A | N/A | mMol/L |
| 3 | Ketone | 0.6 | 3 | N/A | N/A | mMol/L |
| 4 | Ketone | 0.6 | 1.5 | 3 | 5 | mMol/L |
| 5 | Ketone | 1.5 | 3 | 5 | N/A | mMol/L |
| 6 | Ketone | 1.5 | 5 | N/A | N/A | mMol/L |
| 7 | Lactate | 2.5 | 4 | N/A | N/A | mMol/L |
| 8 | Lactate | 2.5 | 5 | N/A | N/A | mMol/L |
| 9 | Lactate | 2.5 | 3.5 | 4 | 5 | mMol/L |
| 10 | Lactate | 3.5 | 5 | N/A | N/A | mMol/L |
| 11 | Lactate | 5 | N/A | N/A | N/A | mMol/L |

Accordingly, for a given analyte one or more configurable thresholds may be set. For example, when detecting blood ketones, a first threshold CT1 may be set at 0.6mmol/L, and a second threshold CT2 may be set at 3mmol/L. When a determined concentration of the ketones in a fluid sample is above CT1, a user-selectable option to display an indication related to the determined concentration of the ketones may be used. When a determined concentration of the ketones in the fluid sample is above CT2, the indication may comprise further information, such as a warning that the determined concentration of the ketones is high.

In some circumstances, it may be desirable to set further configurable thresholds, such as CT3 and CT4 shown in the table.

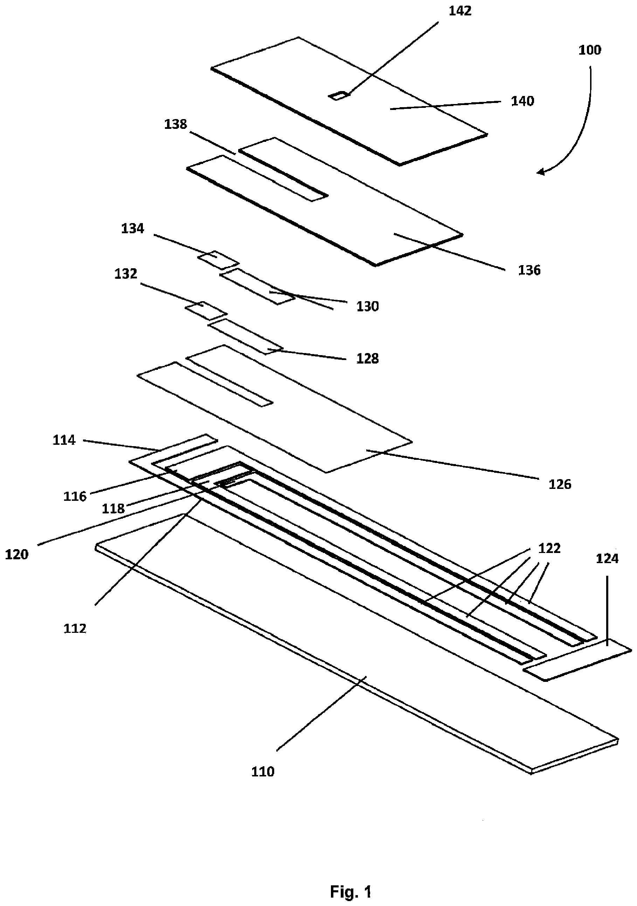

Figure 1 shows a perspective, exploded view of an electrochemical test device in the form of electrochemical test strip 100 according to a first example. This example will be described in relation to a received blood sample of around $0.5\mu l$ in volume, although the electrochemical test strip 100 could be used with any suitable fluid sample. The electrochemical test strip 100 shown in Figure 1 has an end-fill configuration i.e. the blood sample can be received at one end of the electrochemical test device 100. Other architectures to that shown in Figure 1 may be used as will be appreciated by the skilled person.

The electrochemical test strip 100 comprises a support layer or substrate 110. Substrate 110 has a thickness of around 0.35mm. The substrate 110, in this example, is made from polyester, although any suitable substrate may be used. The substrate 110 is thermally and dimensionally stable, with consistent properties such as thickness, surface roughness and surface energy.

Above the substrate 110 is the conductor layer 112. In this example, the conductor layer 112 is directly disposed upon the substrate 110 using carbon-based ink. In this example, the conductor layer 112 is printed directly onto the upper surface of the substrate 110. The conductor layer 112 may be printed onto the substrate 110 using screen printing, lithographic printing, tomographic printing, sub-microlitre controlled volume drop on demand printing technologies, or any other suitable method of printing. The conductor layer comprises a set of electrodes including a first working electrode 114, counter/reference electrode 116, a second working electrode 118 and fill-sufficiency detect electrode 120. The conductor layer 112 further comprises a set of conductive tracks 122. In this example, the conductive tracks 122 extend along the longitudinal axis of the electrochemical test strip 100. The conductive tracks are suitable for electrically coupling the electrodes to an apparatus, or test meter, in order for the test meter to analyse currents produced by target analytes in the blood sample. The conductor layer 112 further comprises a switch-on bar 124 for activating the apparatus or test meter.

Above the conductor layer 112 is an insulator layer 126. The insulator layer 126 is made of an electrically insulating material, and is directly disposed upon the upper surface of the conductor layer 112. The insulator layer 126 is, in this example, made of a dielectric material and defines an interaction area. That is, the insulation layer 126 electrically insulates some portions of the conductor layer 112 from the layers situated above in the electrochemical test strip 100. Specially designed gaps in the insulator layer 126 expose some portions of the conductor layer 112 to the layers situated above in the electrochemical test strip 100. In this way, the insulator layer 126 defines which part or parts of the electrodes of the conductor layer 112 are able to come into contact with an applied blood sample for the measurement of the analyte.

Sensing chemistry is applied to the electrodes of the conductor layer 112. In this example, the sensing chemistry comprises four reagent layers 128, 130, 132 and 134 which are applied to exposed electrode interaction areas after the insulator layer 126 is formed. More or fewer reagent layers may be present. The first working electrode 114 has sensing chemistry (reagent layers 132 and 134) for a first analyte. A first reagent layer 128 is applied to both the second working electrode 118 and the counter/reference electrode 116. In this example, the first reagent layer 128 is also applied to the fill-sufficiency detect electrode 120. An additional analyte-sensitive layer 130 is applied to the second working electrode 118, the counter/reference electrode 116 and the fill-sufficiency detect electrode 120. In this way, the second working electrode 118 is provided with sensing chemistry for a second analyte. The sensing chemistry for the second working electrode 118 comprises suitable reagents for detecting the second analyte.

Although the reagent layers 132 and 134 are not shown to be in contact with the counter/reference electrode 116, they may also be in contact with the counter/reference electrode 116.

Above the insulator layer 126 is a spacer layer 136 formed of a polyester core. The spacer layer 136 defines a sample introduction channel 138, or measurement chamber, for introducing a blood sample to the conductor layer 112. The height of the sample introduction channel 138 is defined by the thickness of the spacer layer 136. The spacer layer 136 is formed of double sided adhesive tape which, in this example, is applied directly to the upper surface of the insulator layer 126. The sample introduction channel 138 is formed by providing a gap into the double sided adhesive tape of the spacer layer 136. The thickness of the spacer layer 136 is approximately 0.1mm, which provides a good balance between the volume of the sample introduction channel 138 and the performance of the electrochemical test strip 100.

Above the spacer layer 136 is a cover layer 140. During manufacture, the spacer layer 136 and the cover layer 140 may be applied to the test strip 100 separately or as a single prelaminated layer, although in this example the cover layer 140 is a separate layer to the spacer layer 136. The cover layer 140 acts as a ceiling to the sample introduction channel 138, thereby substantially closing the sample introduction channel 138 from above. The cover layer 140 is formed of single sided tape and, in this example, is adhered directly to the upper surface of the spacer layer 136. The lower surface of the cover layer 140 has hydrophilic properties, which assist in drawing a blood sample into the sample introduction channel 138. The cover layer 140 further has a vent 142 suitable for venting air out of the sample introduction channel 138 to allow a blood sample to enter the sample introduction channel 138 via capillary action. The vent 142 is narrower than the sample introduction channel 138 so that air may easily vent from the sample introduction channel 138 but blood or any other fluid will not easily be able to pass through the vent 142.

In use, a fluid sample is provided to the electrochemical test device and a potential difference is applied across the fluid sample to generate a detectable output signal indicative of one or more analyte concentrations in the fluid sample. In this example, in use a blood sample is applied to the sample introduction channel 138 of the electrochemical test strip 100. Through capillary action, the blood is drawn into the sample introduction channel 138 to the electrodes 114, 116, 118 and 120 of the conductor layer 112. That is, the sample introduction channel 138 acts as a capillary channel. A potential difference is applied across the electrodes 114 and 116 and the blood sample, and the electrodes 118 and 116 and the blood sample, and output signals such as transient currents are generated from the blood sample. The characteristics of the output signal(s) can be used to determine the concentrations of one or more analytes, such as glucose, lactate or $\beta$-hydroxybutyrate, in the blood sample.

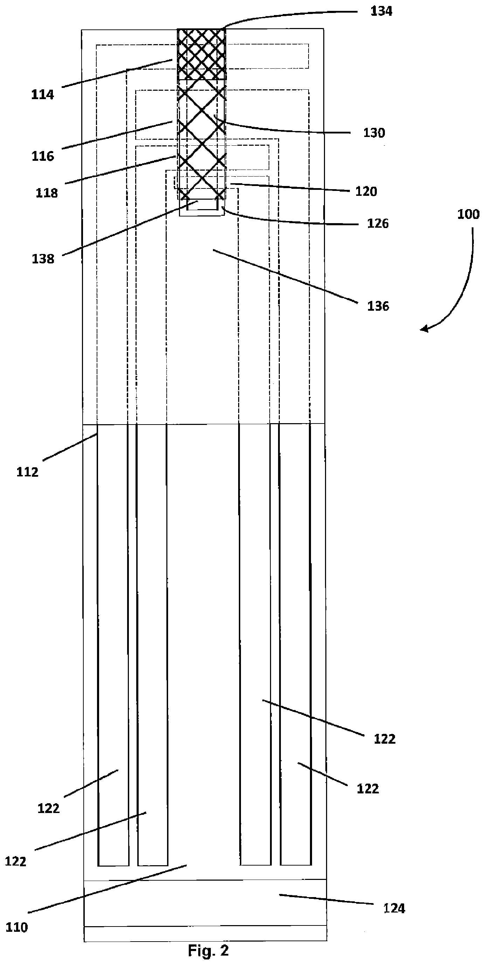

Figure 2 depicts a plan view of some of the layers of the electrochemical test strip 100 of Figure 1. In particular, Figure 2 shows the substrate 110, the conductor layer 112, the insulator layer 126, the reagent layers 130, 134 (reagent layers 128, 132 below are not visible), and the spacer layer 136. The cover layer 140 is not shown in Figure 2 for clarity. The respective reagent layers are applied to the exposed areas of each of the working electrodes 114 and 118, the counter/reference electrode 116 and the fill-sufficiency detect electrode 120.

In the electrochemical test device 100 of Figure 2, the reagent layers 128 and 130 applied to the counter/reference electrode 116, second working electrode 118 and fill-sufficiency detect electrode 120 are shown to be touching the reagent layers 132 and 134 applied to the first working electrode 118. There may be a gap between the sensing chemistry of the counter/reference electrode 116 and the sensing chemistry of the first working electrode 118. The sensing chemistry layers may touch.

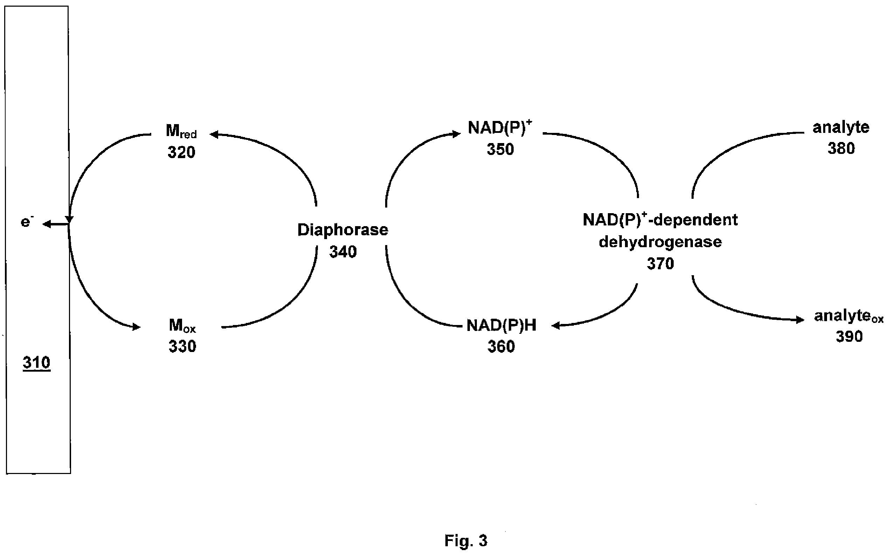

Figure 3 is an illustration of bio-electrocatalysis at an electrode 310, which may correspond to one or more of the working electrodes 114, 118 described above in relation to Figure 1, according to an example. In this example, electrode 310 is coated with layers of sensing chemistry suitable for reacting with an analyte 380 in a fluid sample. The sensing chemistry comprises a cofactor, nicotinamide adenine dinucleotide NAD(P)$^+$ 350, a NAD(P)$^+$-dependent dehydrogenase 370 for reacting with the analyte 380, a diaphorase 340 and an electron transfer agent or mediator (its reduced form $M_{red}$ 320 and its oxidised form $M_{ox}$ 330).

The cofactor 350 may be nicotinamide adenine dinucleotide phosphate (NADP$^+$).

The diaphorase 340 and the entrapped mediator (320, 330) carry out the following reactions:

$$NADH + H^+ + Mediator \rightarrow NAD^+ + Reduced\ Mediator \qquad (1)$$

$$NADPH + H^+ + Mediator \rightarrow NADP^+ + Reduced\ Mediator \qquad (2)$$

Either NADH (the reduced form of NAD$^+$) or NADPH may be used as reductants.

The diaphorase 340 may be any suitable diaphorase. For example, the diaphorase may be an NADPH:acceptor oxidoreductase (NADPH dehydrogenase of the class EC 1.6.99.1). The diaphorase may be an NADH:acceptor oxidoreductase (NADH dehydrogenase of the class EC 1.6.99.3). The diaphorase may be an NADH:(quinone acceptor) oxidoreductase (NADH dehydrogenase (quinone) of the class EC 1.6.99.5).

With reference to Figure 3, when in the presence of a fluid sample, a cofactor NAD(P)$^+$ 350 and a potential difference between the electrode 310 and a counter/reference electrode, an NAD(P)$^+$-dependent dehydrogenase 370 interacts with the analyte 380 of the fluid sample. NAD(P)H 360 is produced as a result of the interaction, as is an oxidised form of the analyte 390. In the presence of a diaphorase 340 (which acts as a catalyst), an oxidised form of a mediator 330 reacts with the NAD(P)H 360 at the active site of the diaphorase 340 to produce a reduced mediator 320 and NAD(P)$^+$ 350. The reduced form of the mediator 320 then transfers electrons (e$^-$) to the electrode 310. In this way, the sensing chemistry applied to electrode 310 accomplishes the transfer of electrons from the fluid sample to the conducting electrode 310. A signal is thus generated to be detected by a strip meter.

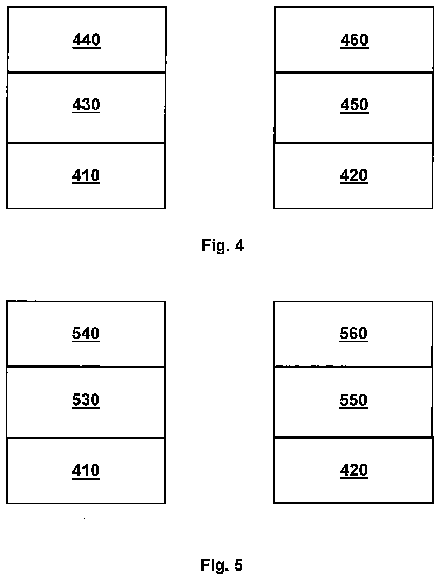

Figure 4 is a schematic showing the respective reagent layers applied to the electrodes of an electrochemical test device according to an example. In Figure 4, a first working electrode 410 for measuring an acidosis-related analyte, and a second working electrode 420 for measuring glucose are shown. In this example, the acidosis-related analyte to be measured is a ketone, specifically β-hydroxybutyrate. The first working electrode 410 may correspond to the first working electrode 114 of Figure 1. Similarly, the second working electrode 420 may correspond to the second working electrode 118 of Figure 1.

In this example, the first working electrode 410 is coated in two layers 430, 440 of reagents. In particular, the reagent layer 430 comprises an electron transfer agent and a diaphorase. Reagent layer 440 comprises suitable reagents for reacting with a ketone in the sample. For example, reagent layer 440 comprises β-hydroxybutyrate dehydrogenase for reacting with β-hydroxybutyrate, and a cofactor for the β-hydroxybutyrate dehydrogenase such as NAD(P)$^+$.

The second working electrode 420 is provided with two layers of sensing chemistry 450, 460. The layer 450 adjacent the second working electrode 420 comprises an electron transfer agent. Reagent layer 460 comprises a glucose oxidase for reacting with glucose in the sample.

Additionally, each layer of the sensing chemistry comprises suitable buffers, surfactants and other additives. For example, the sensing chemistry may comprise one or more of a buffer, a gelling or thickening agent such as hydroxyethyl cellulose (HEC) or other cellulosic polymers, a rheology/viscosity modifier such as silica, flavin mononucleotide (FMN) for stabilising the diaphorase and a surfactant such as Tween 20.

The electron transfer agents at each of the first and second working electrodes may be the same or different. Any suitable electron transfer agent may be used. For example, potassium ferricyanide may be used as an electron transfer agent. For example, ruthenium pentaammine chloride may be used as an electron transfer agent. For example, ruthenium hexaammine trichloride may be used as an electron transfer agent.

Potassium ferricyanide has a number of benefits as a mediator. In particular, potassium ferricyanide is highly water soluble, has a small molecular weight and fast homogeneous and heterogeneous kinetics. Accordingly potassium ferricyanide supports a large analyte measurement range.

Ruthenium pentaammine chloride has a number of benefits as an electron transfer agent. In particular, ruthenium pentaammine chloride has a standard redox potential of approximately -0.08 Volts. The standard redox potential for the NAD(P)$^+$/NAD(P)H couple is approximately -0.315 Volts. Accordingly, ruthenium pentaammine chloride represents an overpotential with respect to the NAD(P)$^+$/NAD(P)H redox couple of approximately 0.235 Volts. In order to achieve the high level of sensitivity that is required to measure, for example, blood ketones such as β-hydroxybutyrate (typically 0.1mM) it is useful to choose a mediator with a low redox potential so that any interference due to the oxidation of opportunist species is reduced. Ruthenium pentaammine chloride is thus a good candidate for use in an electrode for measuring β-hydroxybutyrate. Furthermore, unlike some other compounds such as potassium ferricyanide, ruthenium pentaammine chloride does not react with surface amino acids present in the diaphorase which could lead to a deterioration of enzyme stability and therefore deterioration in electrode performance, and so the shelf life of the electrochemical test device is improved.

Ruthenium hexaammine trichloride has a standard redox potential of approximately 0.1 Volts, which corresponds to an overpotential with respect to the NAD(P)$^+$/NAD(P)H redox couple of approximately 0.415 Volts. Accordingly, ruthenium hexaammine trichloride is a good candidate for use in an electrode for measuring blood ketones such as β-hydroxybutyrate, for which the sensitivity of the electrochemical test device is an issue. Additionally, ruthenium hexaammine trichloride does not react with surface amino acids in the diaphorase which could lead to a deterioration in electrode performance. In addition it is known that this molecule is stable over time, reacting little to, for example, moisture, sunlight, temperatures experienced during manufacture and conditions experienced in storage. Accordingly, the sensitivity of an electrochemical test device incorporating this mediator will not deteriorate rapidly over time.

The sensing chemistry may be applied in layers as illustrated in Figure 5. Figure 5 is a schematic showing a first working electrode 410 and a second working electrode 420, each working electrode having sensing chemistry disposed thereon for detecting a corresponding analyte. In this example, electrode 410 is coated with suitable sensing chemistry for detecting lactate and electrode 420 is coated with suitable sensing chemistry for detecting glucose. The first working electrode 410 may correspond to the first working electrode 114 of Figure 1. Similarly, the second working electrode 420 may correspond to the second working electrode 118 of Figure 1.

In this example, the first working electrode 410 is coated in two layers 530, 540 of reagents. In particular, the reagent layer 530 comprises an electron transfer agent. Reagent layer 540 comprises suitable reagents for reacting with lactate in the sample. For example, reagent layer 540 comprises lactate oxidase for reacting with lactate.

The second working electrode 420 is provided with two layers of sensing chemistry 550, 560. The layer 550 adjacent the second working electrode 520 an electron transfer agent. Reagent layer 560 comprises a glucose oxidase for reacting with glucose in the sample.

The skilled person would appreciate that other variations of the sensing chemistry are possible. For example, there may be one or more reagent layers on each electrode. Layers 530 and 540 may contain both lactate oxidase and a mediator, such as potassium ferricyanide. Similarly, layers 550 and 560 may contain both glucose oxidase and a mediator such as potassium ferricyanide.

As with the example discussed above in relation to Figure 4, each layer of the sensing chemistry may comprise suitable buffers, surfactants and other additives. For example, the sensing chemistry may comprise one or more of a buffer, a gelling or thickening agent such as hydroxyethyl cellulose (HEC) or other cellulosic polymers, a rheology/viscosity modifier such as silica, and a surfactant such as Tween 20.

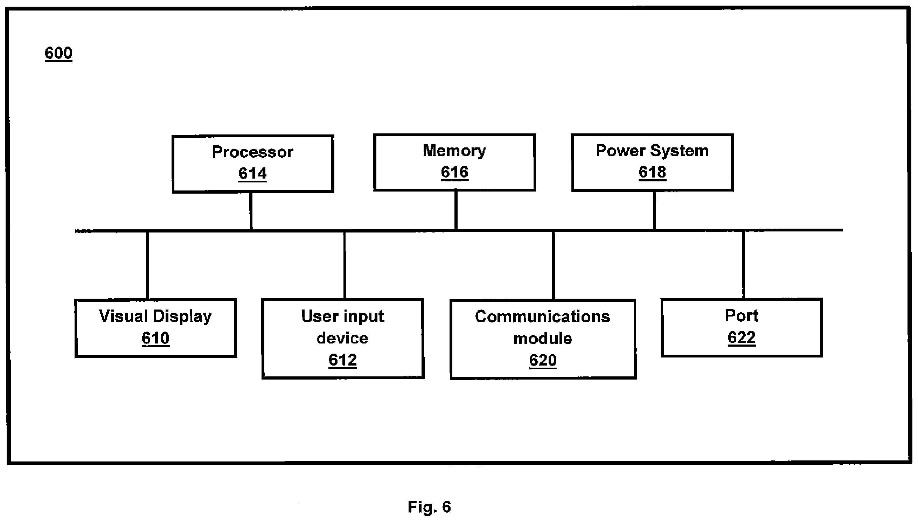

Figure 6 shows an apparatus in the form of a test strip-meter 600 suitable for processing data from one or more electrochemical test devices, such as the multi-analyte electrochemical test strip 100 described above. Other architectures to that shown in Figure 6 may be used as will be appreciated by the skilled person.

Referring to the Figure, the meter 600 includes a number of user interfaces including a visual display 610 and a virtual or dedicated user input device 612. The meter 600 further includes a processor 614, a memory 616 and a power system 618. The meter 600 further comprises a communications module 620 for sending and receiving communications between processor 614 and remote systems. The meter 600 further comprises a port 622 for receiving an electrochemical test device which may be provided with a fluid sample to be analysed.

The processor 614 is configured to receive data, access the memory 616, and to act upon instructions received either from said memory 616, from communications module 620 or from user input device 612. The processor is further configured to control the application of potential differences across electrodes of an electrochemical test device when an electrochemical test device is docked in port 622, to receive output signals, such as transient current data, from the docked electrochemical test device, and to analyse said received output signals. The processor controls the display 610 and may communicate date to remote parties via communications module 620.

In use, a multi-analyte test strip such as electrochemical test strip 100 described above is received by port 622. The processor 614 causes a potential difference to be applied between the fill-sufficiency detect electrode 120 and the counter/reference electrode 116. A fluid sample, in this example a blood sample, is then applied to the electrochemical test strip 100 and flows through the sample introduction chamber 138 via capillary action towards the electrodes 114, 116, 118 and 120. Once a fill-sufficiency detect signal is detected by the processor 614, the fill-sufficiency detect signal indicative of the sample introduction chamber 138 being filled with the blood sample, the potential difference between the fill-sufficiency detect electrode 120 and the counter/reference electrode is turned off. A detection or measurement of one or more analytes in the blood sample can then be undertaken. In this example, the fill-sufficiency detect signal comprises a transient current signals.

The processor 614 controls the application of a potential difference between the first working electrode 114 and the counter/reference electrode 116 and further controls the application of a potential difference between the second working electrode 118 and the counter/reference electrode 116. Reactions between the sensing chemistry of the first working electrode 114 and a first analyte in the fluid sample generate a first output signal indicative of the concentration of the first analyte in the blood sample. Reactions between the sensing chemistry of the second working electrode 118 and a second analyte in the blood sample generate a second output signal indicative of the concentration of the second analyte in the blood sample. The first and second output signals may comprise transient current signals. The first and second output signals are received by the processor 614. The processor may then analyse the first and second output signals to determine a concentration of the first analyte in the blood sample and a concentration of the second analyte in the blood sample. Analysing the first and second output signals may comprise performing operations to account for sources of systematic error. For example, analysing the first and second output signals may comprise applying one or more algorithms to account for errors due to blood sample haematocrit at the first and second working electrodes.

The processor 614 is configured to output an indication of the determined glucose concentration to the display 610 on the test meter 600. If prompted, or in accordance with rules stored in the memory 616, the processor 614 may output an indication of the determined concentration of the first analyte to the display. If prompted, or in accordance with rules stored in the memory 616, the determined data may be communicated to an external device.

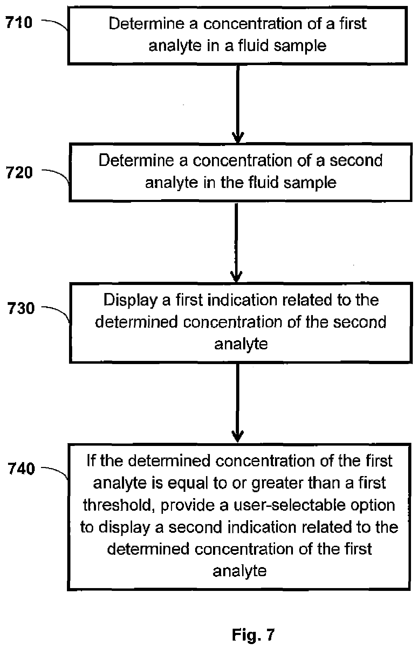

Figure 7 is a flowchart illustrating an example of a method that may be performed by an apparatus such as test meter 600. At step 710, a concentration of a first analyte in a fluid sample is determined using first sensing chemistry. The first analyte is an acidosis-related analyte. As described above, the determination of the concentration of the first analyte in the fluid sample may be made by applying a potential difference across a first working electrode (having first sensing chemistry comprising reagents suitable for reacting with the first analyte to be measured) and a counter/reference electrode of an electrochemical test device. The first sensing chemistry may cause a first output signal such as a transient current to be generated, the first output signal indicative of a concentration of the first analyte in the fluid sample. The first output signal may be processed or analysed by a processor on the apparatus.

At step 720, a concentration of a second analyte in the fluid sample is determined using second sensing chemistry. The second analyte is glucose. As described above, the determination of the concentration of the glucose in the fluid sample may be made by applying a potential difference across a second working electrode (having second sensing chemistry comprising reagents suitable for reacting with glucose in the fluid sample) and the counter/reference electrode of the electrochemical test device. The second sensing chemistry may cause a second output signal such as a transient current to be generated, the second output signal indicative of a concentration of the glucose in the fluid sample. The second output signal may be processed or analysed by a processor on the apparatus.

The skilled person would appreciate that steps 710 and 720 may be performed in any order or at substantially the same time. For example, a determination may be made of the concentration of glucose in the fluid sample before a determination is made of the concentration of the first analyte in the fluid sample.

At step 730, a first indication related to the concentration of glucose in the fluid sample is displayed on user-readable display 610. The indication may comprise, for example, the determined concentration of the glucose in the fluid sample. The indication may comprise a message such as "high" to indicate that the concentration of the glucose in the fluid sample is higher than a glucose threshold and that the user should undertake mitigating or preventative actions to lower the level of glucose in their body. The indication may comprise a message such as "low" to indicate that the concentration of the glucose in the fluid sample is lower than a glucose threshold and that the user should undertake mitigating or preventative actions to raise the level of glucose in their body. The indication may comprise a message such as "normal" to indicate that the concentration of glucose in the fluid sample is neither high enough nor low enough to warrant extraordinary actions - that is, the determined glucose level indicates a euglycaemic condition of the user. The indication may also urge a user to seek medical attention where necessary.

At step 740 a determination is made as to whether the concentration of the first analyte is greater than or equal to a threshold. If the determined concentration of the first analyte is greater than or equal to the threshold then a user-selectable option is provided (step 740), to display a second indication related to the determined concentration of the first analyte. The user-selectable option may be in the form of an icon or button that may be selected by a user via the user input device 612. The user-selectable option may take any suitable form. The user selectable option may comprise, for example, displaying results, a colour indication, an information message, a warning, a recommendation for action, or an audible, vibrational, or wireless message. The user selectable option may comprise, for example, an informative symbol such as an arrow pointing upwards to indicate a high concentration of the first analyte or an increase in the concentration of the first analyte since a previous test was undertaken. An informative symbol may indicate that there has been no change in the concentration of the first analyte since a previous test was undertaken. An informative symbol may indicate that the concentration of the first analyte has decreased since a previous test was undertaken.

If the determined concentration of the first analyte is less than the threshold then no action is taken.

The skilled person would appreciate that, although at step 740, the condition for providing the user-selectable option is that the concentration of the first analyte is greater than or equal to a predetermined threshold, the condition may be that the first analyte is greater than a predetermined threshold. In examples, the method further comprises receiving user-selection of the predetermined threshold.

If both the concentration of the first analyte and the concentration of the second analyte are high then a user may be suffering from an acidosis-related condition such as hyperglycaemic ketoacidosis and may need to seek medical attention. If the concentration of the first analyte is high but the concentration of the second analyte is in the normal range, then a user may be at risk of an acidosis-related condition such as euglycaemic ketoacidosis and may need to seek medical attention.

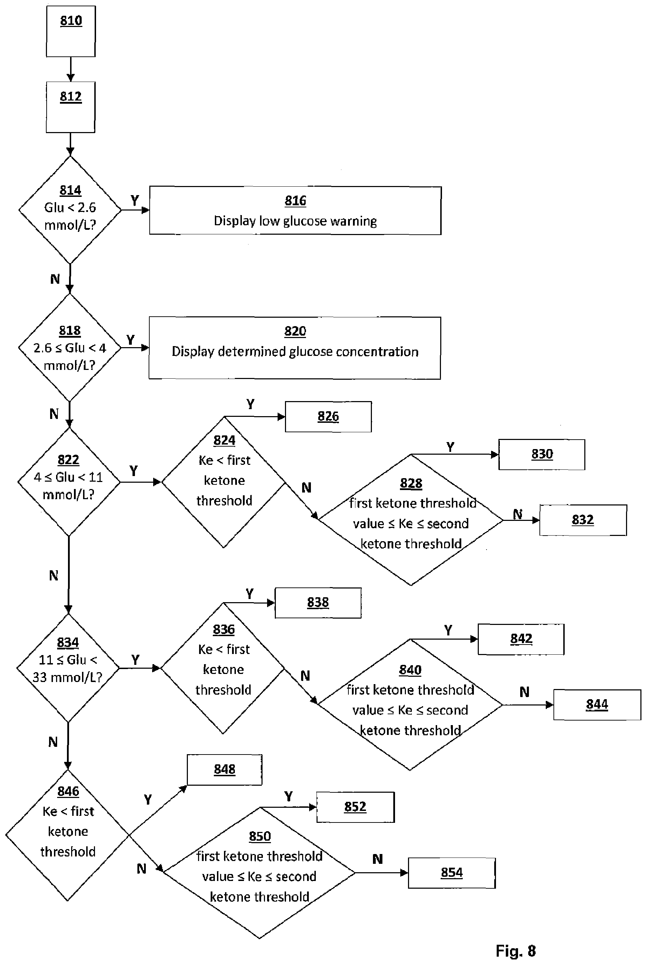

Figure 8 depicts a method according to another example. At step 810 a concentration of a first analyte, in this example a ketone, in a fluid sample is determined. At step 812 a concentration of a second analyte, in this example glucose, is determined. The concentrations of the first and second analytes in the fluid sample may be determined using the methods and apparatuses described above. The first analyte in this example is a ketone but may be any acidosis-related analyte, such as lactate.

At step 814, the determined concentration of glucose is compared against a first glucose threshold value, which in this example is 2.6 mmol/L. If the determined concentration of glucose is less than the first glucose threshold value, then a warning is displayed (step 816), the warning indicating that the concentration of glucose in the fluid is low. If the determined concentration of glucose is greater than or equal to the first glucose threshold value, then the method proceeds to step 818.

At step 818, the determined concentration of glucose is compared against a second glucose threshold value, which in this example is 4 mmol/L. If the determined concentration of glucose is greater than or equal to the first glucose threshold value and less than the second glucose threshold value then the determined glucose concentration is displayed (step 820). If the determined concentration of glucose is greater than or equal to the second glucose threshold value, then the method proceeds to step 822.

At step 822, the determined concentration of glucose is compared against a third glucose threshold value, which in this example is 11 mmol/L. If the determined concentration of glucose is greater than or equal to the second glucose threshold value and less than the third glucose threshold value, then the method proceeds to step 824. At step 824, the determined concentration of the ketone is compared with a first ketone threshold value. The first ketone threshold value is set by the user, optionally, set by a user from a default, pre-programmed value. For this example, it is assumed that the first threshold ketone value is 0.1 mmol/L, although other values may be chosen, for example, 0.6mmol/L. If the determined concentration of the ketone is less than the first ketone threshold value, then the determined concentration of the glucose is displayed (step 826). If, however, the determined concentration of the ketone is greater than or equal to the first ketone threshold value then the method proceeds to step 828.

At step 828, the determined concentration of the ketone is compared with a second ketone threshold value. As with the first ketone threshold value, the second ketone threshold value is set by the user, optionally, set by a user from a default, pre-programmed value. For this example, it is assumed that the second ketone threshold value is 3 mmol/L, although any threshold value, for example 5mmol/L, may be selected. If the determined concentration of the ketone is greater than or equal to the first ketone threshold value and less than or equal to the second ketone threshold value, then the determined concentration of glucose is displayed (step 830). Additionally, a user-selectable option is provided at step 830 for displaying the determined ketone concentration value. If, however, the determined concentration of the ketone is greater than the second ketone threshold value then the method proceeds to step 832. At step 832, the determined concentration of the glucose is displayed. Additionally, at step 832, a user-selectable option is provided for displaying the determined ketone concentration value. Additionally, at step 832, an indication is provided that the ketone concentration value is above the second ketone threshold value, and therefore it may be in the user's interest to select the user-selectable option for displaying the determined ketone concentration value.

Returning to consider step 822, if the determined concentration of the glucose is greater than the third glucose threshold value, then the method proceeds to step 834. At step 834, the determined concentration of the glucose is compared with a fourth glucose threshold value, which in this example is 33 mmol/L. If the determined concentration of the glucose is greater than or equal to the third glucose threshold value and is less than the fourth glucose threshold value then the method proceeds to step 836. At step 836 the determined concentration of the ketone is compared with the first ketone threshold value. If the determined concentration of the ketone is less than the first ketone threshold value, then the determined concentration of the glucose is displayed (step 838). If, however, the determined concentration of the ketone is greater than or equal to the first ketone threshold value then the method proceeds to step 840.

At step 840, the determined concentration of the ketone is compared with the second ketone threshold value. If the determined concentration of the ketone is greater than or equal to the first ketone threshold value and less than or equal to the second ketone threshold value, then the determined concentration of glucose is displayed (step 842). Additionally, a user-selectable option is provided at step 842 for displaying the determined ketone concentration value. If, however, the determined concentration of the ketone is greater than the second ketone threshold value then the method proceeds to step 844. At step 844, the determined concentration of the glucose is displayed. Additionally, at step 844, a user-selectable option is provided for displaying the determined ketone concentration value. Additionally, at step 844, an indication is provided that the ketone concentration value is above the second ketone threshold value, and therefore it may be in the user's interest to select the user-selectable option for displaying the determined ketone concentration value.

Returning to consider step 834, if the determined concentration of the glucose is above the fourth glucose concentration value then the method proceeds to step 846. At step 846 the determined concentration of the ketone is compared with the first ketone threshold value. If the determined concentration of the ketone is less than the first ketone threshold value, then a warning is displayed, the warning indicating that the determined concentration of the glucose is greater than the fourth glucose threshold value (step 848). If, however, the determined concentration of the ketone is greater than or equal to the first ketone threshold value then the method proceeds to step 850.

At step 850, the determined concentration of the ketone is compared with the second ketone threshold value. If the determined concentration of the ketone is greater than or equal to the first ketone threshold value and less than or equal to the second ketone threshold value, then a warning is displayed, the warning indicating that the determined concentration of the glucose is greater than the fourth glucose threshold value (step 852). Additionally, a user-selectable option is provided at step 852 for displaying the determined ketone concentration value. If, however, the determined concentration of the ketone is greater than the second ketone threshold value then the method proceeds to step 854.

At step 854, a warning is displayed, the warning indicating that the determined concentration of the glucose is greater than the fourth glucose threshold value. Additionally, at step 854, a user-selectable option is provided for displaying the determined ketone concentration value. Additionally, at step 854, an indication is provided that the ketone concentration value is above the second ketone threshold value, and therefore it may be in the user's interest to select the user-selectable option for displaying the determined ketone concentration value.

An example system is now described. The example system relates to measurements of β-hydroxybutyrate and glucose for the detection, management and prevention of DKA including euglycaemic DKA. Further examples can alternatively or additionally include measurements of lactate for the detection, management and prevention of lactic acidosis.

When the system is set up initially for the patient a series of 'Background Settings' are configured which inform the system of any likely euglycaemic DKA and/or lactic acidosis risk factors.

In an example, these settings include at least one setting selected from the group comprising:

(i) whether the user is pregnant/ has gestational diabetes;
(ii) whether the user is prescribed insulin, and optionally the dose taken, the type of insulin (fast acting, long acting, single dose, multiple daily injections, pump user etc), as well as optionally the insulin sensitivity factor of the individual;
(iii) whether the user is prescribed SGLT2 medication (and optionally the dose taken);
(iv) whether the user is prescribed metformin or SGLT2 inhibitor metformin combination therapy;
(v) whether the user suffers from alcohol dependency;
(vi) whether the user suffers from an eating disorder such as anorexia or bulimia (including diabulimia);
(vii) whether the user is following a carbohydrate restricted diet; and
(viii) whether the user has previously experienced a DKA episode in the absence of elevated blood glucose.

In an example, when DKA is considered a risk and ketones and glucose are simultaneously measured, if a positive response is elicited to any of questions (i), (iii), (iv), (v), (vi) or (vii) above, the system shall subsequently operate in a manner which is consistent with a sustained higher risk of euglycaemic DKA.

If a negative response is elicited to all of the questions except (ii), the system operates in a manner which is consistent with no sustained higher risk of euglycaemic DKA.

System behaviour in normal use - where previous 3 tests have not suggested DKA

Whenever a test is performed, in all instances the glucose result will be communicated to the user immediately after completion of the test. Thereafter, the subsequent response from the system to the user is governed according to the following algorithms, based upon the measured ketone and glucose results.

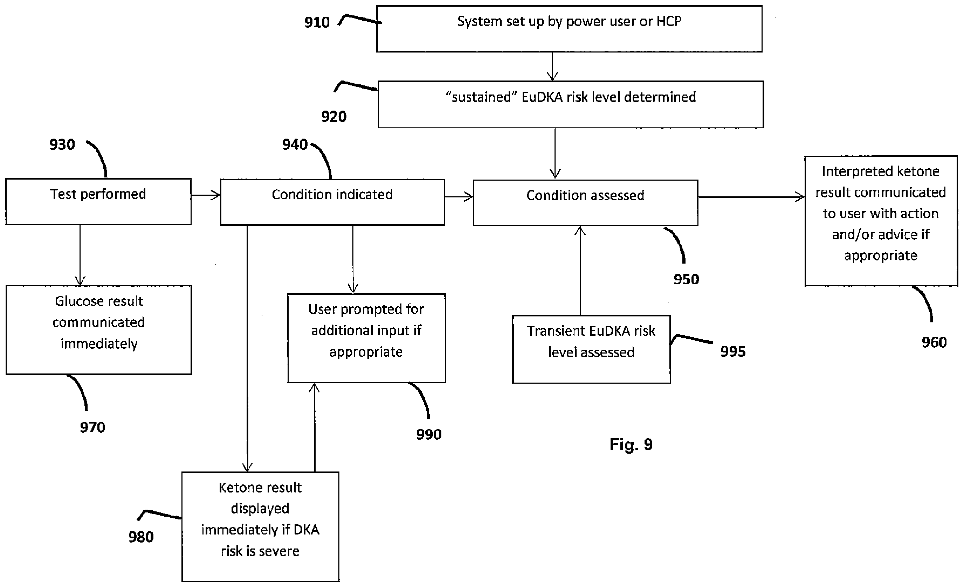

A high level process flow is shown in Figure 9. At step 910 the system is set up by a power user. In this case the power user can be any technically competent person with respect to both the operation of the companion health management tool, and with regard to awareness of and responsibility for the underlying medical wellbeing and treatment of the user and their condition. This power user could therefore be a Health care Professional, a suitably knowledgeable carer, a technician, or a 'non-naïve' user of the system, who is competent in and takes responsibility for actively managing their treatment and condition themselves (e.g. making self-dose adjustments to medication), or a consensus represented by some combination of these individuals. At step 920, a determination is made as to whether or not there is a sustained risk of euglycaemic DKA.

At step 930 a fluid sample test is performed. At step 970 an indication representative of a determined glucose concentration is communicated to the user. At step 940 a "condition indicated" is determined internally by the system but not communicated to the user. The 'condition indicated' is determined based upon configurable thresholds set by the user and the analyte measurements determined in the test performed. One illustrative example of how the 'condition indicated' may be derived from the results of 930 and the thresholds set is shown in the "Condition Indicated" Boolean logic table shown subsequently.

At step 980, an indication, (including for example a warning) related to a determined concentration of ketones in the fluid sample is displayed if the "condition indicated" at 940 corresponds with a severe risk of DKA. Otherwise either the user will be prompted for additional input at step 990, if the "condition indicated" at step 940 determines that such additional user input is warranted, or else a "condition assessed" may be determined directly. A non-limiting function of the additional information input from 990 is to allow a risk of transient euglycaemic DKA (or "transient DKA risk") to be assessed at step 995, in those cases where the "condition indicated" at 940 corresponds to a situation where such a 'transient DKA risk' determination is deemed to be necessary. For example if initially the "Condition Assessed" (950) logic initially produced a result which was "uncertain" in the absence of this supplementary input, then the additional input (990) and "transient DKA risk" (995) steps would be instigated by the system. At step 950 a "condition assessed" is determined internally by the system, based upon the combination of the 'condition indicated' at step 940 (from the analyte readings), as well as from the "sustained euglyclaemic DKA risk level" determined at 920 and in addition, if appropriate, ultimately from the 'transient euglycaemic DKA risk' which may by then have been assessed at step 995. One illustrative example of how the 'Condition Assessed' may be derived from the "Condition Indicated" (940) and the user specific information (910, 920 and if appropriate 990, 995) is shown in the "Condition Assessed" Boolean logic tables shown subsequently:

"CONDITION INDICATED" BOOLEAN LOGIC TABLE

As previously stated, in the first instance, a "condition indicated' is determined, for example according to the 'Condition Indicated' Boolean logic table shown below:

| Glucose value | Ketone value | Condition Indicated |
|---|---|---|
| <4 mmol/L | Any | suspect hypoglycaemia |
| >=4 mmol/L<11.1 mmol/L | <CT1 | euglycaemia |
| >=4 mmol/L<11.1 mmol/L | >=CT1, <CT2 | suspect Euglycemic mild DKA risk |
| >=4 mmol/L<11.1 mmol/L | >=CT2, <CT3 | suspect Euglycaemic DKA risk moderate |
| >=4 mmol/L<11.1 mmol/L | >=CT3 | suspect Euglycemic DKA risk severe |
| >=11.1 mmol/L | <CT1 | suspect hyperglycaemia |
| >=11.1 mmol/L | >=CT1, <CT2 | suspect DKA risk, mild |
| >=11.1 mmol/L | >=CT2, <CT3 | suspect DKA risk, moderate |
| >=11.1 mmol/L | >CT3 | suspect DKA risk, severe |

In this case CT1 represents a first user definable, or configurable threshold concentration for example 0.6 mmol/L, below which concentrations are considered at a level which the user would not be too concerned about. Similarly CT2 represents a user definable, or configurable, threshold above CT1 but below which the user should wish to be informed of the ketone concentration, so as to take avoiding action with the aim of preventing a complication from developing (e.g. <1.5 mmol/L for β-hydroxybutyrate) and above which the user should aim to treat the complication that has developed or is developing. CT3 represents a third user definable, or configurable, threshold above which the complication has reached a point where immediate acute treatment and/or emergency action may be warranted, for example 3 mmol/L for β-hydroxybutyrate.

In the case of Lactate rather than β-hydroxybutyrate, and example threshold for CT1 would be 2.5 mmol/L, and an example setting for CT2 would be 3.5 mmol/L, and an example setting for CT3 would be 5 mmol/L.

The result reported to the user and the recommended advice they are given by the system will subsequently be arrived at on the basis of the 'Condition Assessed', which in turn is arrived at on the basis of the combination of the 'Condition Indicated' (940) along with the background information contained within the meter from initial set up (910, 920), and in some circumstances also in conjunction with the determination of any increase in 'transient risk' of (euglycaemic) DKA (995), which is suggested from responses to appropriate user prompts (990).

In the event that the user's immediately previous 1-3 test results all indicate that DKA risk was not present, an example of how the "Condition assessed" will initially be determined is shown in the on the following table.

"CONDITION ASSESSED" BOOLEAN LOGIC TABLE: NO IMMINENT DKA RISK ALREADY PRESENT (950)":

| Condition Indicated | Risk of euglycaemic DKA determined | Condition assessed |
|---|---|---|
| suspect hypoglycaemia | any | hypoglycaemia |
| euglycaemia | any | euglycaemia |
| suspect euglycaemic mild DKA risk | sustained high risk | euglycaemic mild DKA risk |
| suspect euglycaemic mild DKA risk | no sustained high risk | uncertain - determine transient risk symptoms |
| suspect euglycaemic moderate DKA risk | sustained high risk | euglycaemic moderate DKA risk |

(continued)

| Condition Indicated | Risk of euglycaemic DKA determined | Condition assessed |
|---|---|---|
| suspect Euglycaemic moderate DKA risk | no sustained high risk | uncertain - determine transient risk symptoms |
| suspect Euglycaemic severe DKA risk | no sustained high risk | uncertain - determine transient risk symptoms |
| suspect hyperglycaemia | any | hyperglycaemia |
| suspect DKA risk, mild | any | DKA risk mild |
| suspect DKA risk, moderate | any | DKA risk, moderate |
| suspect DKA risk, severe | any | DKA risk, severe |

In the event that the condition initially assessed is uncertain, it is then that the 'transient' risk of DKA will subsequently be determined by the system on the basis of user inputs (990, 995) in response to prompts such as questions on symptoms.

In the event that the condition indicated is 'mild or moderate DKA risk' and 'no sustained high risk' of DKA was previously identified, the 'transient risk' of DKA will be determined in the first instance (990, 995) by the system through user inputs in response to prompts, before any ketone results and advice are communicated to the user (960).

However, in the event that the condition indicated is severe DKA risk, the ketone result will first be communicated immediately to the user (980), before any transient risk of DKA is determined and appropriate advice subsequently communicated to the user (995, 960).

Determination of Transient DKA Risk (995)

Wherever appropriate, the transient risk of DKA will be assessed by the system based on the user's response to on screen prompts or questions such as in the following examples:

Q1. Have you been in a state of fast (no calorific intake) for the previous 6 hours OR have you been in a state of carbohydrate intake restriction (<~100g Carbohydrate) for the previous 24 Hours? (Y/N)
Q2. Have you consumed and excess of alcohol within the previous 24 hours (Y/N)
Q3. Have you suffered from nausea, vomiting, abdominal pain, shortness of breath, excessive urination, or mental confusion within the previous 12 hours (Y/N)
Q4. Have you been undertaking sustained vigorous exercise / exertion within the previous 3 hours? (Y/N)

In particular, Q4 above is intended to determine whether a user has undertaken anaerobic exercise which may be a cause of increased lactic acid.

An affirmative response to any of these questions results in the determination that there is a positive 'transient elevated risk of DKA or EuDKA' (995). A negative response to all of these questions results in a determination that there is negative transient elevated risk of DKA or Euglycaemic ketoacidosis (995).

In examples, the condition which had initially been assessed as "uncertain" is then revised as follows:

"CONDITION ASSESSED" BOOLEAN LOGIC TABLE: NO SUSTAINED DKA RISK-WHERE INITIAL "CONDITION ASSESSED" WAS "UNCERTAIN":

| Condition Indicated | Transient DKA risk assessed | Sustained DKA risk assessed | Condition Assessed | Action |
|---|---|---|---|---|
| suspect euglycaemic mild DKA risk | negative | negative | possible spurious result | Prompt user to repeat test. If result is repeated, assess as mild Euglycaemic DKA risk |
| suspect euglycaemic mild DKA risk | positive | negative | mild Euglycaemic DKA risk | advise, log and report accordingly |

(continued)

| Condition Indicated | Transient DKA risk assessed | Sustained DKA risk assessed | Condition Assessed | Action |
|---|---|---|---|---|
| suspect Euglycaemic moderate DKA risk | negative | negative | possible spurious result | Prompt user repeat test. If result is repeated, assess as moderate Euglycaemic DKA risk |
| suspect Euglycaemic moderate DKA risk | positive | negative | Euglycaemic moderate DKA risk | advise, log and report accordingly |
| suspect Euglycaemic severe DKA risk | negative | negative | uncertain - determine transient risk symptoms | Prompt user repeat test. If result is repeated, assess as moderate Euglycaemic DKA risk |
| suspect Euglycaemic severe DKA risk | positive | negative | Euglycaemic severe DKA risk | advise and report accordingly |

System behaviour in "DKA management mode" - where one or more of the previous 3 tests have suggested DKA is possible:

In the event that any of the previous three results indicated that DKA risk was already present the Condition Assessed will be determined using different logic, for example as follows:

"CONDITION ASSESSED" BOOLEAN LOGIC TABLE: IMMINENT DKA RISK WAS ALREADY PRESENT":

| Ketone | Glucose | Condition assessed |
|---|---|---|
| <CT1 | >4 mmol/L <= 11.1 mmol/L | DKA management - Ketones absent |
| <CT1 | >11.1 mmol/L | DKA management-Hyperglycaemia risk |
| >CT1 | >4 mmol/L | DKA management - Ketones present. |

Results reporting and advice to user:

Based on the "condition assessed", the system will report and advise the user appropriately, for example as follows

| Condition assessed | Insulin user? | Results Reporting | results logging | Advice to user |
|---|---|---|---|---|
| hypoglycaemia | any | Glucose only | Glucose and Ketone | Take on carbohydrates |
| euglycaemia | any | Glucose, Ketone user selectable | Glucose and Ketone | No action |
| suspect hyperglycaemia | any | Glucose, Ketone always | Glucose and Ketone | Take Action to lower Blood glucose |
| DKA management - Hyperglycaemia | any | Glucose, Ketone always | Glucose and Ketone | Take Action to lower Blood glucose |
| DKA management - Ketones absent | any | Glucose, Ketone always | Glucose and Ketone | No action |
| DKA management - Ketones present. | yes | Glucose, Ketone always | Glucose and Ketone | Follow sick day rules/ DKA management advice |

(continued)

| Condition assessed | Insulin user? | Results Reporting | results logging | Advice to user |
|---|---|---|---|---|
| DKA management Ketones present | no | Glucose, Ketone always | Glucose and Ketone | rehydrate and seek medical advice |
| possible spurious result | any | Glucose, Ketone only if >3mmol/L | Glucose and Ketone | retest |
| DKA risk mild | yes | Glucose, Ketone | Glucose and Ketone | take insulin, rehydrate |
| DKA risk, mild | no | Glucose, Ketone | Glucose and Ketone | rehydrate, seek medical advice if unwell |
| DKA risk, moderate | yes | Glucose, Ketone | Glucose and Ketone | take insulin, rehydrate, seek medical attention if feeling unwell |
| DKA risk, moderate | no | Glucose, Ketone | Glucose and Ketone | Rehydrate, seek medical advice. |
| DKA risk, severe | yes | Glucose, Ketone | Glucose and Ketone | take insulin, rehydrate, and seek medical attention urgently. |
| DKA risk, severe | no | Glucose, Ketone | Glucose and Ketone | rehydrate and seek urgent medical attention |
| euglycaemic mild DKA risk | yes | Glucose, Ketone | Glucose and Ketone | rehydrate, take in carbohydrate, with insulin. |
| euglycaemic mild DKA risk | no | Glucose, Ketone | Glucose and Ketone | rehydrate, take in carbohydrate. |
| euglycaemic moderate DKA risk | yes | Glucose, Ketone | Glucose and Ketone | rehydrate, take in carbohydrate with insulin. Seek medical advice if unwell. |
| euglycaemic moderate DKA risk | yes | Glucose, Ketone | Glucose and Ketone | rehydrate, take in carbohydrate. Seek medical advice if unwell. |
| euglycaemic severe DKA risk | any | Glucose, Ketone | Glucose and Ketone | Seek urgent medical advice attention |

The example logic employed here and the use in combination of the analyte measurements, the persistent risk factors specific to the individual and entered upon meter set up, and the transient risk factors optionally entered in response to user prompting, by which the system is able to progress from the 'condition indicated' state to the 'condition assessed' state demonstrates means by which the annoyance of spurious alerts may be more easily avoided whilst ensuring genuine alerts may be more readily identified.

This specific logic described is by way of example only, and other algorithms of this type can readily be envisaged by those skilled in the art.

The example above describes one specific illustration pertaining to the scenario with respect to ketoacidosis, especially diabetic ketoacidosis including euglycaemic ketoacidosis, based upon appropriate configurable thresholds, persistent risk factors and transient risk factors pertaining to that specific condition and known aetiologies.

The present disclosure is not limited to ketoacidosis associated with diabetes, and other causes of ketoacidosis might also be managed with similar methodology and appropriate algorithms, for example those susceptible to alcoholic ketoacidosis or those ketoacidoses associated with eating disorders.

Comparable logic can be employed in the case of lactic acidosis by use of a combined lactic acid and glucose sensor for the management of lactic acidosis, for example Metformin Associated Lactic Acidosis (MALA), based upon appropriate configurable thresholds, persistent risk factors and transient risk factors pertaining to that specific condition and its known aetiologies.

Furthermore, disambiguation between lactic acidosis (eg MALA) and diabetic ketoacidosis can be accomplished where both lactate and ketones are simultaneously assessed, and the appropriate recommendations presented based on whichever aetiology is determined on the basis of medications and measurements.

Companion Health Management system

The present disclosure further provides a companion health management system. The Companion Health Management system creates and maintains a time stamped log of, for example, the following parameters for each test:

1 Glucose result
2. Ketone result
3. Condition Assessed
4. Whether transient risk was absent, present or not assessed.

Trending and Medication management with Companion Health Management system:

The results log may then be used by the Companion Health Management system to make ongoing recommendations based on best practice guidelines stored therein, and to thereby assist users and their health care providers (HCPs) to adjust therapy accordingly.
For example the following table shows example recommendations that may be made to the user by the system on the basis of results stored within the result log triggering accomplished in the example given above, by looking at the data from across a suitable review period, accessed by means of a 'review history' menu option:
In some examples, this is achieved by the system looking at the weekly, fortnightly or monthly averages and events logs compared across for example, a 3 week, 6 week or 3 month period, to establish the trends, conditions assessed, medications being taken and the risk factors (transient or sustained) present for the individual user.
In the case of the individual lay user, the health management system may output a cursory message which while appropriate may be of limited complexity and low informational burden, since this would be the most appropriate system response to that situation.
Beneath this high level message however, there may also be the option to review in detail all of the results, the conditions assessed, the user responses entered at the time (e.g. whether fasting or emesis was present) and event flags, such that an advanced user or HCP could spot underlying causes among the detail that was automatically captured by the Companion Health Management system at the time tests were performed (and which otherwise would be lost to the vagaries and subjectivity of the user's own recollection) and make recommendations accordingly.

| Medications used | Triggering Parameters | | | | | | | Recommendation made by meter (and suspected underlying cause) |
|---|---|---|---|---|---|---|---|---|
| | Hypo glycaemia | Hyper glycaemia | DKA | EuDKA | Mean glucose | Glucose Trend | Ketones trending | |
| SGLT2 only no insulin | absent | absent | absent | absent | at target | stable | absent/ stable | Affirmation of existing treatment regimen and status **"Well done", "Good management", "You're doing great" etc** (on target) |
| | absent | absent | absent | absent | above target | stable or increasing | absent/ stable | **"consider increase SGLT2 dose"** (no hypoinsulinaemia, but inadequate glycaemic control: insulin resistance?) |
| | absent | present | absent | absent | above target | decreasing | absent/ stable | **Encouragement to continue "Heading in the right direction"** (inadequate glycaemic control, but being corrected ) |
| | absent | present | absent | absent | at or above target | stable or increasing | absent/ stable | consider increase SGLT2 dose **"Are you remembering to take your SGLT2? Is your dose adequate?"** (no hypo insulinaemia but severely inadequate glycaemic control: insulin resistance? Possible hypoinsulinaemia onset, but not yet severe enough to cause problems) |
| | present | any | any | any | any | any | any | **"Something is not** |

| | Triggering Parameters | | | | | | | Recommendation made by meter (and suspected underlying cause) |
|---|---|---|---|---|---|---|---|---|
| Medications used | Hypo glycaemia | Hyper glycaemia | DKA | EuDKA | Mean glucose | Glucose Trend | Ketones trending | |
| | | | | | | | | quite right! Time for a Doctor visit?"<br><br>seek HCP advice (should not experience hypo if not on insulin. suspect carbohydrate insufficiency?) |
| | absent | absent | absent | absent | at target | decreasing | absent stable | consider reduced SGLT2 dose (possible over medication)<br><br>**"Are you still taking your SGLT2 correctly? Is your dose too high?"** |
| | absent | absent | absent | absent or present | at or below target | stable or downward | present or trending upwards | **"Are you eating properly? "Are you still taking your SGLT2 correctly? Is your dose too high?"**<br><br>consider reduced SGLT2 dose (hypoinsulinaemia caused by low glucose. Need to increase glucose to restore insulin levels) |
| | absent | present or absent | present or absent | present or absent | above target | stable or increasing | present or trending upwards | **"Your meds don't seem to be working very well for you. Time for a Doctor's visit!"** hypoinsulinaemia risk<br><br>seek HCP advice consider starting insulin therapy (hypoinsulinaemia caused by insulin secretion |
| | | | | | | | | insufficiency) |
| | absent | present or absent | present or absent | present | any | downwards | present / upwards | **"Are you still taking your SGLT2 correctly? Is your dose too high?"** consider decrease SGLT2 dose (onset of hypoinsulinaemia due to reduced secretion) |

EP 3 310 925 B1

22

(continued)

| | Triggering Parameters | | | | | | | Recommendation made by meter (and suspected underlying cause) |
|---|---|---|---|---|---|---|---|---|
| Medications used | Hypo glycaemia | Hyper glycaemia | DKA | EuDKA | Mean glucose | Glucose Trend | Ketones trending | |
| Insulin only, no SGLT2 | present | absent | absent | absent | at or below target | stable or downwards | absent | **"Review your hypos: Is your insulin dose too high?"**<br><br>consider decrease insulin dose (Hypoglycaemia caused by insulin over treatment) |
| | absent | present | absent or present | absent | above target | stable or increasing | present | **"Review your glucose numbers: Is your insulin dose too low?" "Has you insulin sensitivity factor changed?"**<br><br>consider increase in insulin dose (hypoinsulinaemia caused by poor insulin secretion) |
| | present | present | absent | absent | above target | stable or increasing | absent | **"Are you still taking your insulin correctly? Is your dose too high?" "Should you be taking oral meds as well?"**<br>seek medical advice - (hyperglycaemia related to insulin insensitivity or gluconeogenesis rather than hypoinsulinaemia. |
| | | | | | | | | consider potential to augment insulin with SGLT2 or metformin) |
| | absent | absent | absent | absent | at target | stable | absent/ stable | Affirmation of existing treatment regimen and status **"Well done", "Good management", "You're doing great" etc** (on target) |

EP 3 310 925 B1

23

(continued)

| Medications used | Triggering Parameters | | | | | | | Recommendation made by meter (and suspected underlying cause) |
|---|---|---|---|---|---|---|---|---|
| | Hypo glycaemia | Hyper glycaemia | DKA | EuDKA | Mean glucose | Glucose Trend | Ketones trending | |
| Insulin and SGLT2 both prescribed | absent | absent | absent | absent | at target | stable | absent/ stable | Affirmation of existing treatment regimen and status **"Well done", "Good management", "You're doing great"** etc (on target) |
| | absent | absent | absent | absent | above target | stable or increasing | absent/ stable | **"consider increase in insulin and/or SGLT2 dose"** (no hypoinsulinaemia, but inadequate glycaemic control: insulin resistance?) |
| | absent | present | absent | absent | above target | decreasing | absent/ stable | **Encouragement to continue "Heading in the right direction"** (inadequate glycaemic control, but being corrected ) |
| | absent | present | absent | absent | at or above target | stable or increasing | absent/ stable | consider increase SGLT2 dose and / or insulin dose **"Are you remembering to take your SGLT2 and Insulin? Is your dose adequate?"** (no hypo |
| | | | | | | | | insulinemia but severely inadequate glycaemic control: insulin resistance? Possible hypoinsulinaemia onset. |
| | present | any | any | any | any | any | any | **"Review hypos: Too much insulin?"** |

| | Triggering Parameters | | | | | | | Recommendation made by meter (and suspected underlying cause) |
|---|---|---|---|---|---|---|---|---|
| Medications used | Hypo glycaemia | Hyper glycaemia | DKA | EuDKA | Mean glucose | Glucose Trend | Ketones trending | |
| | absent | absent | absent | absent | at target | decreasing | absent stable | consider reduced SGLT2 dose (possible over medication) **"Are you still taking your SGLT2 correctly? Is your dose too high?" "Do you need to change balance of insulin to SGLT2?**" |
| | absent | absent | absent | absent or present | at or below target | stable or downward | present or trending upwards | **"Are you eating properly? "Are you still taking your SGLT2 correctly? Is your dose too high?" "Are you getting enough insulin?"** consider reduced SGLT2 dose consider increased insulin dose (hypoinsulinaemia caused by low glucose caused by SGLT2. Need to increase glucose and restore insulin levels) |
| | absent | present or absent | present or absent | present or absent | above target | stable or increasing | present or trending upwards | **"Are you getting enough insulin?"** hypoinsulinaemia risk seek HCP advice consider starting insulin therapy (hypoinsulinaemia caused by insulin secretion insufficiency) |
| | absent | present or absent | present or absent | present | any | downwards | present / upwards | **"Are you still taking your SGLT2 correctly? Is your dose too high?"** |

EP 3 310 925 B1

25

(continued)

| Medications used | Triggering Parameters | | | | | | | Recommendation made by meter (and suspected underlying cause) |
|---|---|---|---|---|---|---|---|---|
| | Hypo glycaemia | Hyper glycaemia | DKA | EuDKA | Mean glucose | Glucose Trend | Ketones trending | |
| | | | | | | | | consider decrease SGLT2 dose and **increase** in insulin dose (hypoinsulinaemia due to reduced secretion) |
| | present | absent | absent | absent | at or below target | stable or downwards | absent | **"Review your hypos: Is your insulin dose too high?"**<br><br>consider decrease insulin dose (Hypoglycaemia caused by insulin over treatment) |
| | absent | present | absent or present | absent | above target | stable or increasing | present | **"Review your glucose numbers: Is your insulin dose too low?"**<br><br>consider increase in insulin dose (hypoinsulinaemia caused by poor insulin secretion) |
| | present | present | absent | absent | above target | stable or increasing | absent | **"Are you still taking your insulin correctly? Is your dose too high?" "Should you be taking oral meds as well?"** seek medical advice - (hyperglycaemia related to insulin insensitivity or gluconeogenesis rather than hypoinsulinaemia. consider potential to augment insulin with SGLT2 or metformin) |

The above schema gives one example of a Companion Health Management tool that uses the conditions assessed and the measurements made and medication type to provide substantially optimal ongoing management to an example user.

In this case, the system has the capability to differentiate appropriately between for example the following cases, and in each case advise accordingly:

(a) A type I diabetic needing insulin to avoid DKA & Hyperglycaemia, but at doses that allow the user to avoid hypoglycaemia. The optimum balance of insulin and other medications is the goal of this user's management, with the minimisation of the risk of hyperglycaemia and hypoglycaemia, whist ensuring that there is enough insulin to stay clear of DKA episodes.

(b) A type II diabetic on oral medications who is prone to hyperglycaemia caused by insulin insensitivity. This user has adequate insulin and will not be prone to DKA. The optimal treatment may be glycaemic control by SGLT2 and / or other orals along with diets and exercise aimed towards managing blood glucose downwards and reducing the extent of insulin insensitivity, while maintaining insulin secretion levels in as near a normal state as possible. Upwardly regulating their SGLT dosages for the maximum benefit may be the appropriate management for this user.

(c) A type II diabetic on oral medications who was in condition (b) but is now progressing from insulin insensitivity towards insulin insensitivity with concomitant reduced insulin secretion. This user is at a great risk of hyperglycaemia and will be prone to sudden DKA onset and even euglycaemic DKA if they control their blood glucose aggressively by medication and / or diet and exercise in the absence of adequate insulin. Over aggressive treatment with SGLT2 and other oral medications may push this user in the direction of hypoinsulinaemia and DKA. Appropriate management for this user is a step down in the SGLT2 therapy, either in terms of dose level or dose frequency, with a possible eventual switch to or commencement of insulin therapy, or combined insulin/oral therapy being the best management approach ultimately.

(d) A type 1 diabetic who could benefit from adjunctive SGLT2 inhibitor therapy in combination with insulin to better maintain glycaemic control within the optimum range.

Alternate management schema which are based on lactate results and lactic acidosis aetiologies can be similarly derived by those skilled in the art in light of the present disclosure. Concentrations of analytes in a fluid sample may be provided to a user in any suitable units. For example, a concentration may be provided in units of mg/dL. For example, a concentration may be provided in units of mmol/L.

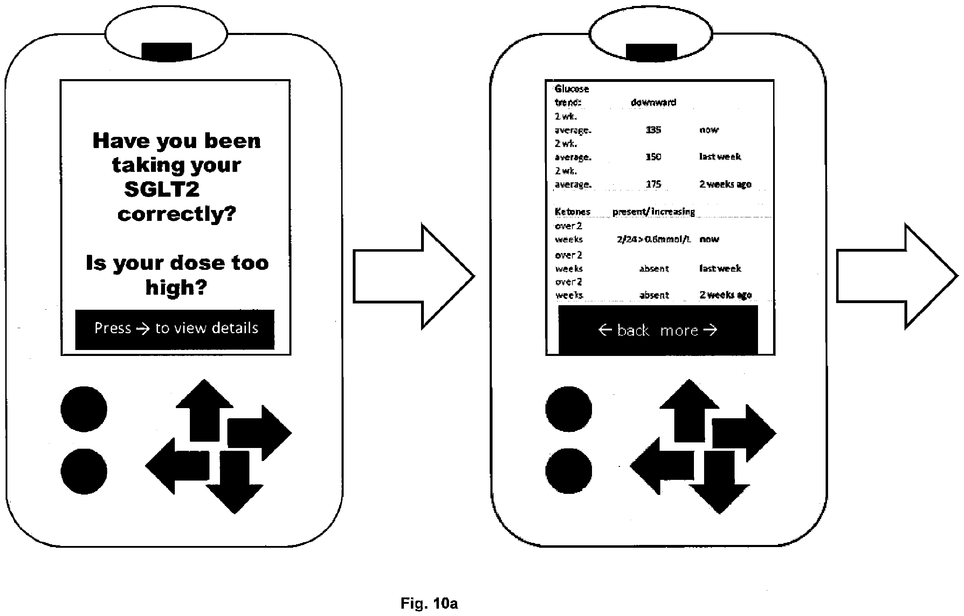

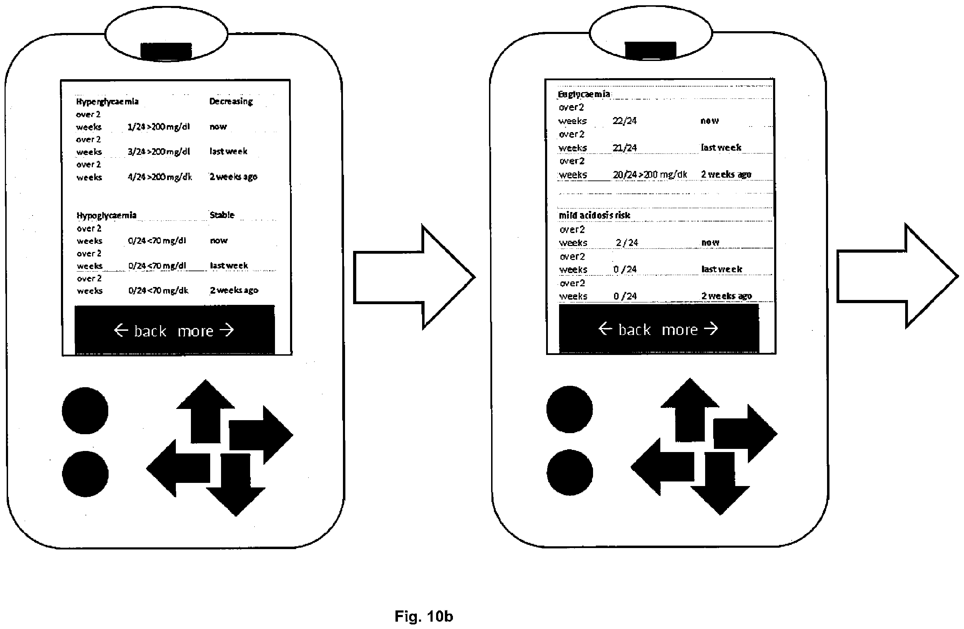

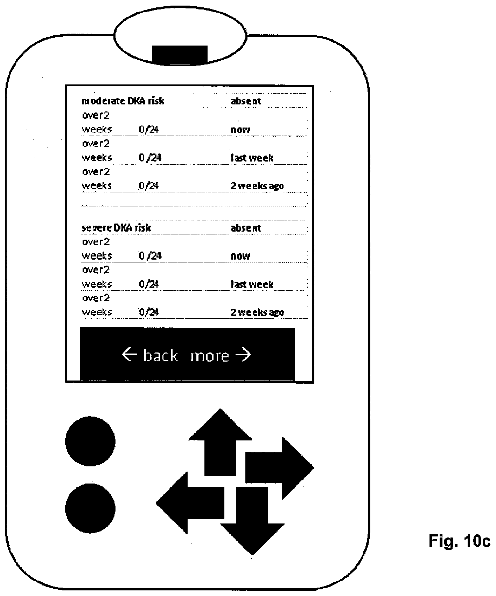

Figures 10a, 10b and 10c show example screen shots on such a companion health management tool. In Figure 10a, a user is presented with a display asking the user whether the user has been taking their SGLT2 correctly, and whether the dosage is too high. The user is prompted to view details of recent measurements. On the second screen, the measured glucose trend and the ketone trend are displayed. The user is presented with an option to go back to the first screen or to proceed to a third screen with further information. On selecting to proceed with the third screen, the user is provided with a display of information as to whether they are at risk of hyperglycaemia or hypoglycaemia based on recent trends (Fig. 10b). The user is given an option to return to a previous screen or to proceed to a fourth screen. On the fourth screen, the user is provided with information as to how many measurements indicated euglycaemia, and any measurements that indicated a mild acidosis risk. The user is provided the option to go back to a previous screen or to proceed to a fifth screen (Fig. 10c). On the fifth screen, the user is provided with information pertaining to any recent measurements indicative of a moderate DKA risk or a severe DKA risk.

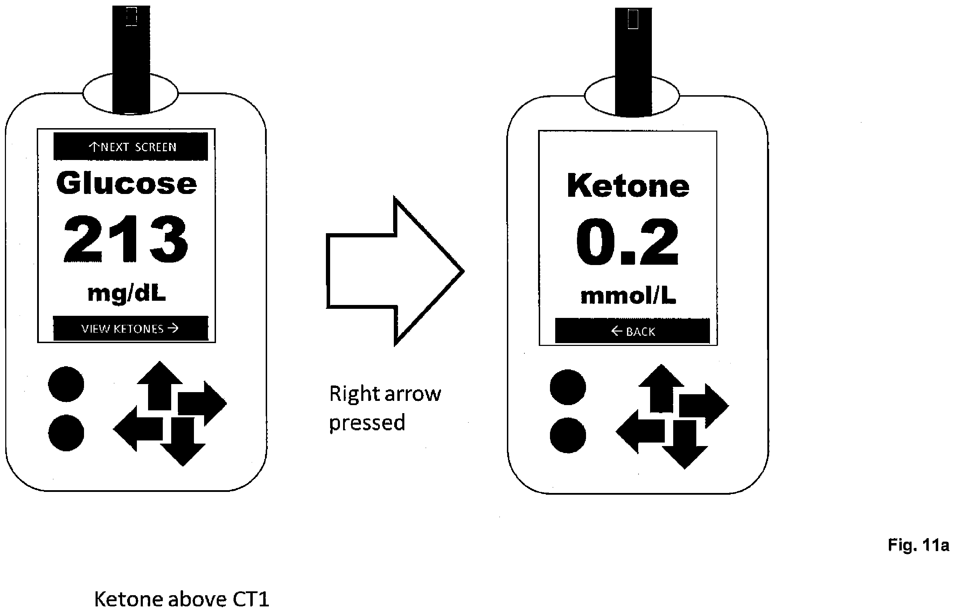

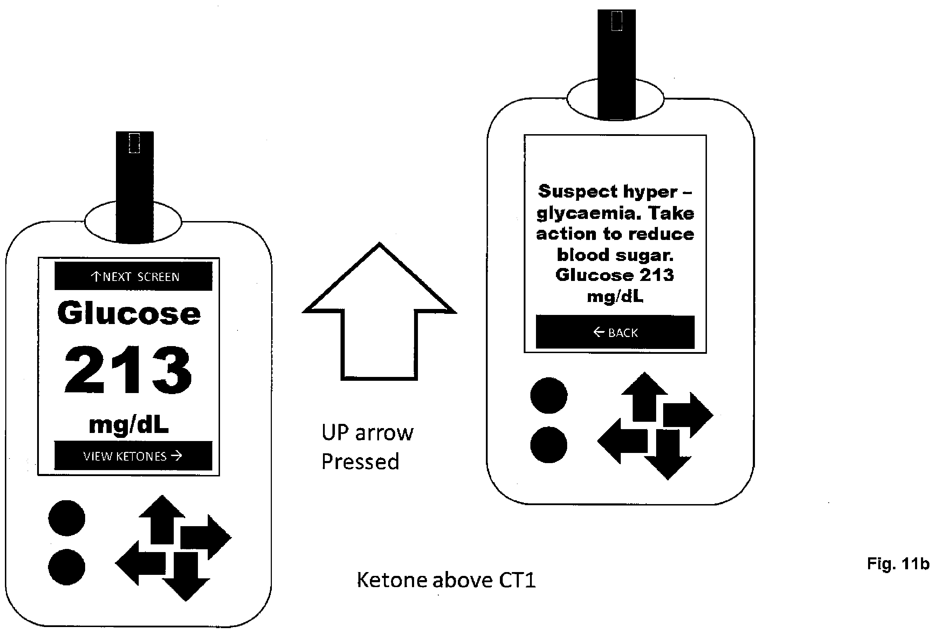

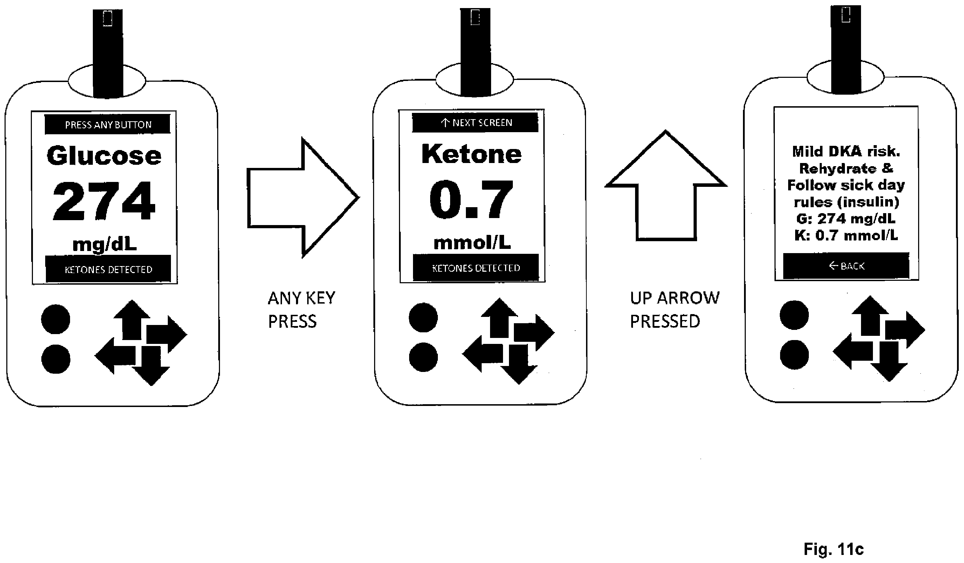

Figures 11a, 11b and 11c show example screen shots when measurements are made to determine a concentration of glucose and to determine a concentration of a ketone in a fluid sample. As shown in Figure 11a, on a first screen a first indication related to the determined concentration of the glucose is provided. In this example, the first indication is the concentration, 213 mg/dL, of the glucose in the fluid sample. A determined concentration of the ketone in the fluid sample is greater than or equal to a first threshold, CT1, and so a user-selectable option is provided to display a second indication related to the concentration of the ketone in the fluid sample in response to a specific key press. In this example, the ketone first threshold CT1 is 0.1 mmol/L. If the user-selectable option is selected by the user, the determined concentration of the ketones is displayed. In this example, the determined concentration of the ketone is displayed as 0.2 mmol/L. A user-selectable option to return to the previous screen is also provided (see Fig. 11b). On the first screen a further user-selectable option is also provided. In this example, the further user-selectable option is a prompt to press an up arrow on a keypad. On selection of the further user-selectable option, a further screen is displayed providing advice to the user. In this example the measurement of the glucose concentration is indicative of hyperglycaemia and so a message is displayed.

The message suggests to the user that the user take action to reduce the concentration of glucose in their body.

Fig. 11c shows further examples of screen shots. In a first screen shot a first indication related to the determined concentration of glucose is displayed. As the determined concentration of ketones in the fluid sample is above a user-definable threshold CT1, in this example 0.1 mmol/L, a user-selectable option is provided to display a second indication related to the determined concentration of ketones in the fluid sample, in response to any key press. In this example, the second indication is a determined concentration of the ketones in the fluid sample. A further user-selectable option to display further information is provided. On selection of the further user-selectable option, the user is presented with information related to the determined concentrations of glucose and ketone and is further presented with advice, for example actions that the user may undertake in order to stave off potential health issues. In this example, the determined glucose concentration of 274 mg/dL and the determined ketone concentration of 0.7 mmol/L indicate a mild risk of DKA. The user is advised to rehydrate and to follow sick day rules concerning insulin.

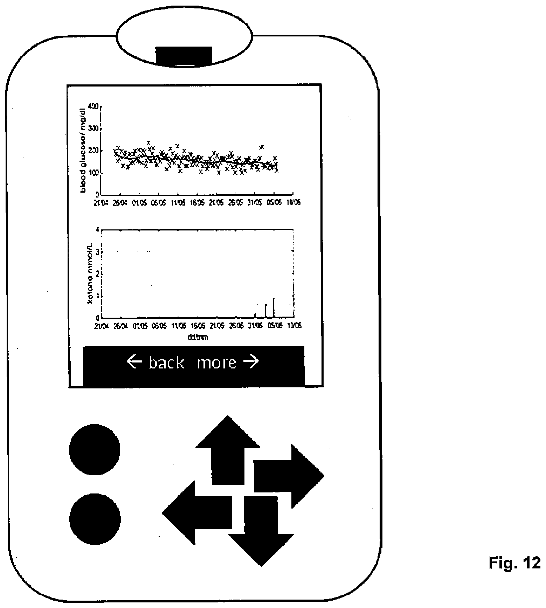

Figure 12 shows a screen shot of trending information that may be shown. In particular, a graph of recent glucose measurements is shown. In particular a graph of recent ketone measurements is also shown.

Another condition that can be considered either in addition to or as an alternative to the scenario described above relates to lactic acidosis instead of ketoacidosis, and specifically to lactic acidosis where MALA may be a consideration.

In this situation the "Condition indicated" and/or "Condition Assessed" may be the existence or onset of MALA.

Alternatively, the conditions indicated and/ or condition assessed may be suspected lactic acidosis induced by sepsis or ischemia, or alternatively, benign transient lactate elevation caused by strenuous exercise.

The "sustained risk factors" assessed for MALA in this situation would include, for example: biguanide based medications such as metformin, phenformin, or buformin, along with any other indications or patient history that could be indicative as to the possibility of impaired renal function, such as kidney function test results to cite one example.

The transient risk factors for MALA in this situation could include, for example, dehydration and emesis and such other factors as could be indicative of acute renal impairment.

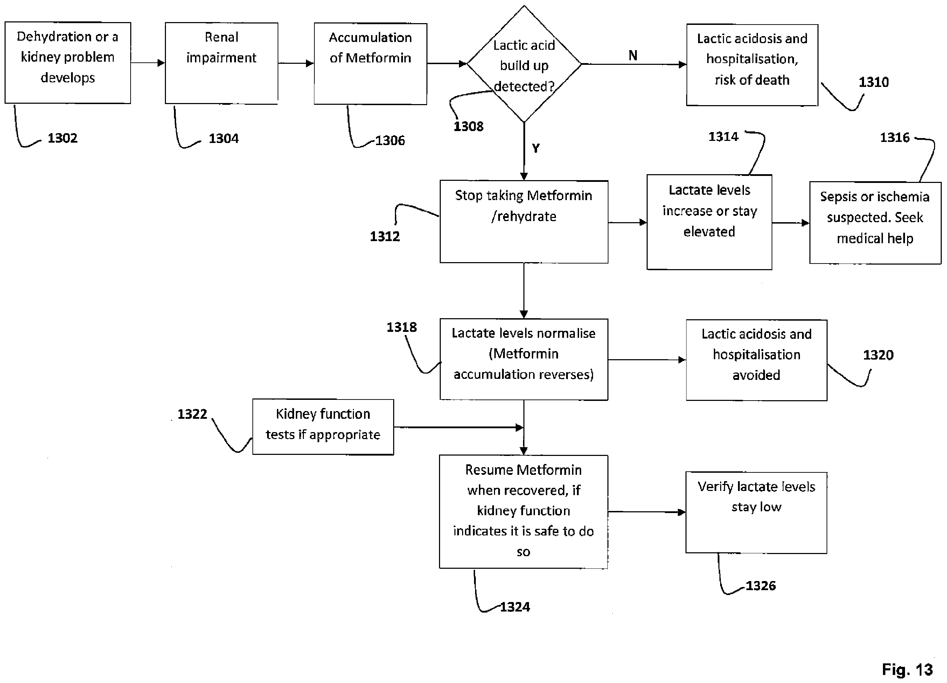

The flow chart in Figure 13 shows the benefit that could be accomplished by exploiting an example of the present disclosure. Figure 13 shows the development/prevention of MALA in Metformin treated patients. At step 1302, dehydration or a kidney problem may develop, leading to renal impairment (1304). Accordingly, Metformin may accumulate in a patient (1306) leading to a build-up of lactic acid (1308).

In the event of undetected Metformin Associated Lactic Acidosis (MALA), hospitalisation is almost inevitable and a serious risk of death is present (1310).

With appropriate use of the present disclosure as described, disambiguation between MALA and sepsis or ischemia related acidosis becomes facile, and appropriate and timely treatment for whichever condition is actually identified (at an intervention level which may be far below that of the Emergency Room) becomes enabled.

If a lactic acid build-up is detected then appropriate action may be taken. For example, if a concentration of lactic acid is above a threshold then a user may be provided with an indication related to the determined concentration of lactic acid. Configurable thresholds for Lactate (CT1, CT2, CT3) could be set at, for example, 2.5 mmol/L, 3.5 mmol/L and 5 mmol/L respectively, depending upon the individual user's metabolism and risk profile.

In this case, the transient risk factors, user prompts and selectable user inputs would be configured with logic such that elevated lactate produced in a benign fashion in the muscles merely as a response to vigorous exertion that was dissipated quickly (within a few hours) was readily identified and disambiguated from elevated lactate that was the result of a more sinister cause. This could be effected by means of establishing the presence of a sustained elevation of or upward trend in lactate in the absence of exertion through, for example, prompting the user to conduct timely follow up testing after a period of rest or low intensity exertion, for example as outlined in the flow chart in Figure 13.

For example, if a lactic acid build-up is detected at step 1308, then a user may stop taking Metformin and/or rehydrate (1312). If the user's lactate levels increase or remain elevated (1314) then sepsis of ischemia may be suspected a medical treatment may be sought (1316).

The potential onset of MALA and its disambiguation from, for example, ischemia or sepsis related Lactic acidosis (both of which would necessitate their own appropriate care and management) would be established through the absence of evidence of sepsis or ischemia related symptoms (e.g. chest pains, breathlessness, infection, etc.) after the user was prompted to identify the presence or absence of such symptoms in the situation where elevated lactate readings had been detected by the system, as well as by the trend towards normalisation of lactate levels that followed on from the (possibly temporary) cessation of bigunaide (e.g. metformin) therapy. This could be accomplished by identifying an upward trend or sustained elevation in lactate level in the absence of exertion, rather than relying on a single high reading that subsequently reduced when the user was sedentary. Additionally by advising users to at least temporarily desist from their metformin therapy, MALA could potentially be avoided altogether and the underlying cause of the lactic acidosis within that user (e.g. cumulative build-up of biguanides due to inadequate excretion of these by the kidneys) thereby more readily identified.

If, after desisting from taking Metformin and/or rehydrating, a user's lactate levels normalise (1318) then lactic acidosis and hospitalisation may be avoided (1320). After kidney function tests if appropriate (1322) to determine that a user's kidney functions have returned to normal, a user may resume taking Metformin (1324) and tests may be taken over time to verify that the user's lactate levels stay low (1326).

Example symptoms and conditions indicative of ischemia or sepsis that the system would prompt the user to highlight when moving from the 'condition indicated' to the 'condition assessed' determination, and in accomplishing the disambiguation of the acidosis type could include, for example:

- known or obvious sepsis (sepsis)
- known ischemia (ischemia)
- high temperature (sepsis)
- localised oedema or wounds (sepsis)
- localised pain or irritation (sepsis)
- tachycardia/ breathlessness (ischemia or sepsis)
- chest pain (ischemia)
- sudden fatigue (ischemia or sepsis)

Example "Sustained" risk factors for MALA would include:

Metformin or other Biguanide use
Known renal impairment
Kidney test results and metrics

Transient risk factors for MALA would include:

Severe Dehydration (leading to renal insufficiency)
Time since last urination (indicating renal impairment and / or severe dehydration)
Symptoms consisted with renal abnormality (e.g. back pain, general skin itchiness)
Exclusion of ketonuria (by blood or urine test) or negative blood ketone test (Lactate in presence of ketones would instead indicate likely ketoacidosis, albeit with a greater severity than ketones alone would suggest, for which rehydration and insulin therapy would be indicated).
Absence of known sepsis and sepsis related symptoms
Absence of recent vigorous exertion / exercise
Absence of known ischaemia and ischemia related symptoms

"Conditions assessed" by the system thereby would include, for example:

Normal: Euglycaemia and lactate below CT1;
Hypoglycaemia: Any SMBG reading <4 mmol/L;
Hyperglycaemia: →based upon blood glucose readings in the absence of elevated lactate;
Possible DKA risk: (no elevated lactate in the presence of ketonurea or elevated blood ketones and high blood glucose readings);
Severe DKA risk → elevated lactate in the presence of ketonurea or elevated blood ketones and high blood glucose readings;
Exertion based lactate → transient lactate elevation which trends towards normal after a sedentary period;
Sepsis or ischemia based Lactic acidosis → elevated lactate which is sustained or increasing in the absence of exertion, in the absence of metformin therapy, or which does not reverse upon metformin discontinuation and/or exists in the presence of other ischemia or sepsis indications; and
Possible MALA onset: elevated lactate levels in the absence of sepsis or ischemia symptoms which corrects upon discontinuation of metformin.

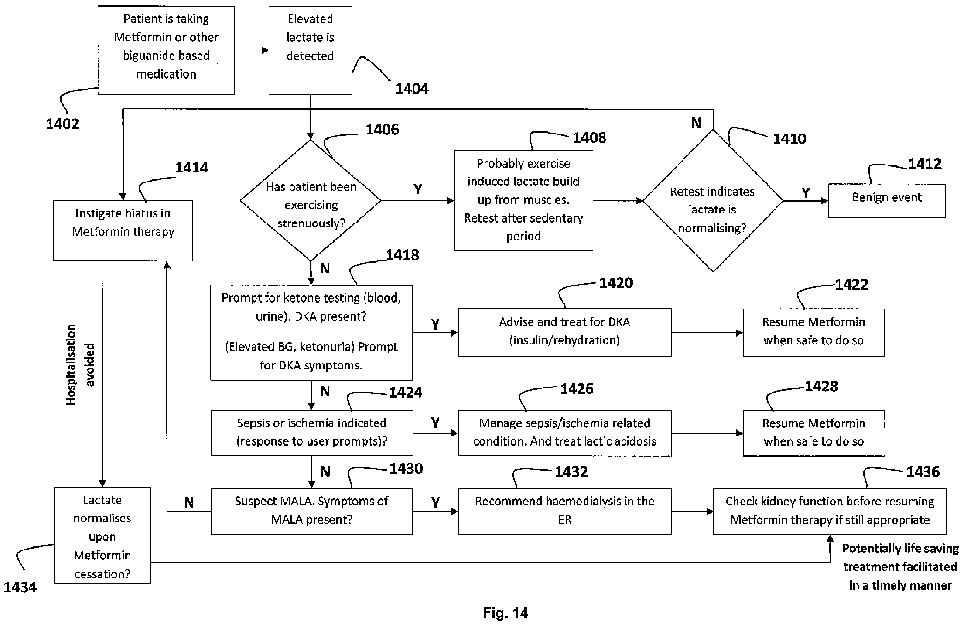

A process flow such as that shown in Figure 14 illustrates the procedural steps that the system would work through in arriving at the appropriate condition assessed.

During set up of the system, at step 1402, an indication is made by a power user or health care provider as to whether the patient or user is taking Metformin or another biguanide-based medication.

In the event that a test detected an elevated lactate level (1404) the patient or user is invited to indicate whether or not they have performed recent strenuous anaerobic exercise (1406). If the user indicates that they have performed recent strenuous exercise, then there is a possibility that the elevated lactate result of step 1404 is the result of anaerobic respiration arising from physical exertion. In the incidence of recent physical exertion being confirmed by the user, a retest after a sedentary period is prompted (1408). A retest is performed (1410) and if this retest indicated a trend towards normalisation of lactate levels, the elevated lactate reading is disregarded as a benign instance of anaerobic respiration (1412).

If a retest at step 1410 indicates that the user's lactate levels are not normalising, then at step 1414 then the user instigates a hiatus in the Metformin or other biguanide-based therapy. If the user's lactate levels normalise after this (step 1434) then the user's kidney functions are checked (1436) before resuming Metformin or other biguanide-based medication.

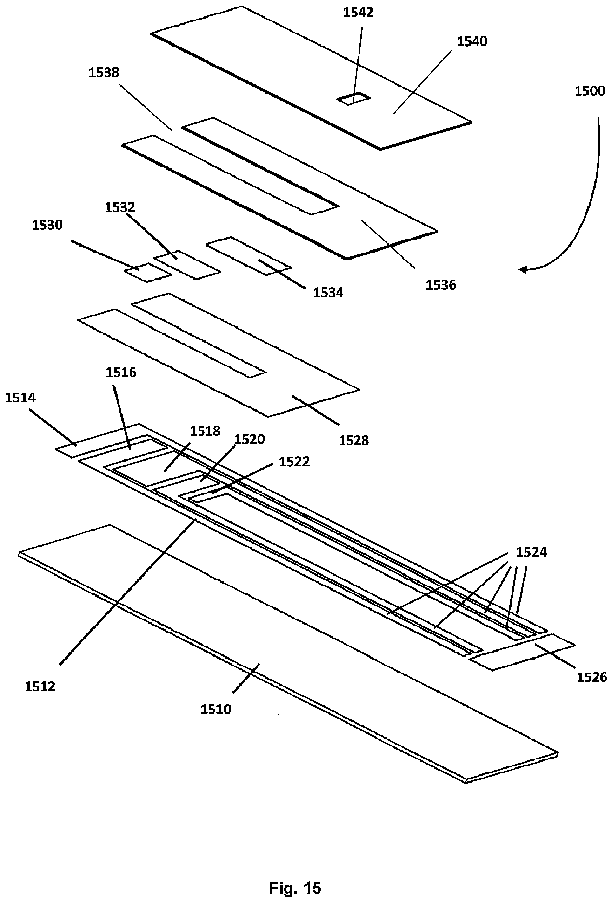

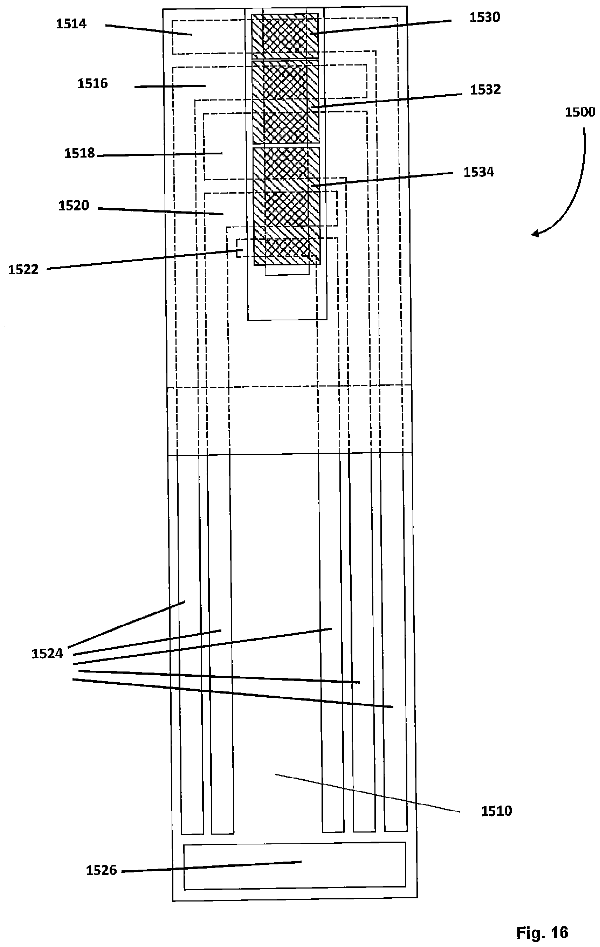

If at step 1406 the user indicated that they had not undertaken recent anaerobic exercise the user would be prompted to assess for DKA by means of blood ketone or urine ketone measurement (1418). When using some example test devices, such as those discussed below in relation to Figures 15 and 16, substantially simultaneous measurements of ketones and lactate concentrations are made and so no further ketone test is required.

DKA is considerably more common and can be less severe than MALA, and so in the event that ketones are detected, a DKA episode would be assessed. This condition could be managed accordingly (1420) by, for example, following sick day rules and seeking an appropriate level of medical intervention in a timely fashion. Upon successful resolution of the DKA episode, metformin therapy could be resumed (1422).

In the absence of ketones being detected (1424) then an indication may be provided that the user is at risk of sepsis or ischemia. The indication may be provided in response to the user inputting evidence or symptoms consistent with these conditions when prompted to do so. If a sepsis or ischemia related acidosis is assessed, then the user could be prompted to seek appropriate treatment and management accordingly (1426) prior to resuming any Metformin based or other biguanide based treatment (1428).

In the further absence of sepsis or ischemia systems, biguanide based acidosis is suspected (1430). The user is prompted to identify appropriate symptoms and their severity (1430) and the user instigates a hiatus in the Metformin or other biquanide-based therapy (1414). In the event that symptoms were severe, haemodialysis in the Emergency Room is recommended (1432) in an appropriate and timely fashion. The user's kidney functions are checked (1436) before resuming Metformin or other biguanide-based medication. In the event that MALA onset was mild or caught early and that levels showed signs of normalisation after the cessation of biguanide therapy (1434), then severe MALA and hospitalisation could be avoided altogether, and the user could be prompted to seek kidney function tests in a primary healthcare setting (1436) which would establish whether biguanide therapy was still appropriate for them, before it was resumed.

Figure 15 shows a perspective, exploded view of an electrochemical test device in the form of electrochemical test strip 1500 according to an example. The electrochemical test strip shown in Figure 15 is formed of layers in much the same way as the electrochemical test strip of Figure 1. As with the electrochemical test strip 100 of Figure 1, this example will be described in relation to a received blood sample of around $0.5\mu l$ in volume, although the electrochemical test strip 1500 could be used with any suitable fluid sample. The electrochemical test strip 1500 shown in Figure 15 has an end-fill configuration.

The electrochemical test strip 1500 of Figure 15 comprises a support layer or substrate 110. Above the substrate 1510 is the conductor layer 1512, which comprises a first working electrode 1514 for sensing chemistry for detecting a first analyte, a second working electrode 1516 for sensing chemistry for detecting a second analyte, a counter/reference electrode 1518, a third working electrode 1520 for sensing chemistry for detecting a third analyte and a fill-sufficiency detect electrode 1522. Each of the electrodes is connectable to an apparatus or test meter via conductive tracks 1524. The conductor layer 1512 further comprises a switch-on bar 1526. Above the conductor layer 1512 is an insulator layer 1528 which defines an interaction area for the electrodes in the conductor layer 1512. Sensing chemistry (reagent layers 1530, 1532 and 1534) is then applied to the exposed areas of the electrodes. Above the insulator layer 1528 is a spacer layer 1536 having a sample introduction channel 1538. Above the spacer layer 1536 is a cover layer 1540 having a vent 1542.

In this example, the first working electrode 1514 is provided with sensing chemistry 1530 for detecting an acidosis-related analyte such as the ketone body β-hydroxybutyrate. The second working electrode 1516 is provided with sensing chemistry 1532 for detecting a second analyte, such as glucose. The third working electrode 1520 is provided with sensing chemistry 1534 suitable for detecting an acidosis-related analyte such as lactate.

Any of the reagent layers 1532, 1534, 1536 may comprise an electron transfer agent, or mediator. In an example, reagent layer 1530 comprises an electron transfer agent having a low standard redox potential such as ruthenium pentaammine chloride or ruthenium hexaammine trichloride. Reagent layers 1534 and 1536 may comprise an electron transfer agent such as potassium ferricyanide.

The reagent layers may comprise any suitable reagents for detecting the respective analytes for each of the working electrodes. For detecting a ketone body such as β-hydroxybutyrate, sensing chemistry 1530 for first working electrode 1514 may comprise β-hydroxybutyrate dehydrogenase, a cofactor, and a diaphorase. For detecting glucose, sensing chemistry 1532 for second working electrode 1516 may comprise glucose oxidase. For detecting lactate, sensing chemistry 1534 for third working electrode 1520 may comprise lactate oxidase.

In use, a fluid sample is provided to the electrochemical test device and a potential difference is applied across the fluid sample to generate a detectable output signal indicative of one or more analyte concentrations in the fluid sample. In this example, in use a blood sample is applied to the sample introduction channel 1538 of the electrochemical test strip 1500. Through capillary action, the blood is drawn into the sample introduction channel 1538 to the electrodes 1514, 1516, 1518, 1520, and 1522 of the conductor layer 1512. That is, the sample introduction channel 1538 acts as a capillary channel. A potential difference is applied across the electrodes 1514 and 1518 and the blood sample, and the electrodes 1518 and 1516 and the blood sample, and the electrodes 1520 and 1516 and the blood sample, and output signals such as transient currents are generated from the blood sample. The characteristics of the output signal(s) can be used to determine the concentrations of one or more analytes.

Figure 16 depicts a plan view of some of the layers of the electrochemical test strip 1500 of Figure 15. In particular, Figure 16 shows the substrate 1510, the conductor layer 1512, the insulator layer 1528, the reagent layers 1530, 1532, 1534, and the spacer layer 1536. The cover layer is not shown in Figure 16 for clarity. The respective reagent layers are applied to the exposed areas of each of the working electrodes 1514, 1516 and 1520, the counter/reference electrode 1518 and the fill-sufficiency detect electrode 1522.

Examples where both lactate and β-hydroxybutyrate measurements are effected simultaneously enable more ready and immediate disambiguation of the acidosis type using appropriate modifications of the logic already described.

Also, because lactate is often also present in cases of ketoacidosis , and the rarer lactic acidosis is likely to be the underlying cause and condition in circumstances where ketones are absent and only lactate is present, lactic acidosis is more likely to be correctly identified as the "condition indicated" within these tri-analyte systems.

Since -68% of DKA episodes include some concomitant lactate elevation, the combined lactate/ketone result from the tri-analyte system can be used to better assess the severity and appropriate management of any ketoacidosis present (where ketones are detected and lactate is present or absent).

For example in circumstances where ketone results were normalising but lactate was continuing to trend upwards, a DKA management regimen or sick day rules that might have otherwise been incorrectly abandoned prematurely would be persisted with by the user, such that acute DKA management was only ended when all 3 levels (glucose, lactate, ketone) had been restored to acceptable levels.

In another example, a DKA episode with a moderate ketone level of e.g. 1mmol/L with an elevated lactate of e.g. 8 mmol/L that may have been assessed as minor or moderate in the case of glucose and ketone data alone (and therefore appropriate for self-management), could be more appropriately elevated to 'severe DKA risk' within the tri-analyte system, once the highly elevated lactate result was factored in along with the ketone and glucose readings. Thus leading to an appropriate and timely health care intervention that could otherwise have been in appropriately delayed.

For example, the severity of an acidosis event could be more appropriately determined by the aggregate (or a suitable weighted aggregate) of the combined analytes above their respective CT1 threshold values, instead of solely on the basis of a single analyte above its individual CT1 threshold value.

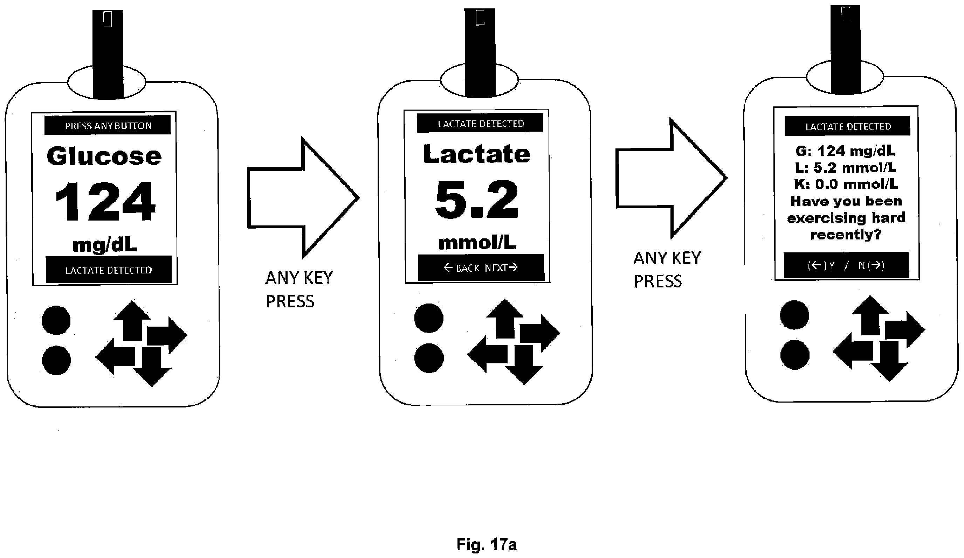

Figures 17a-17d show example screenshots on an apparatus or test meter when measurements are made to determine a concentration of glucose, a concentration of lactate and a concentration of ketone in a fluid sample.

In Figure 17a, an indication of a determined glucose concentration is displayed in a first screen. A determined concentration of lactate is above a threshold and so a user selectable option is provided to the user (in this example a prompt to press any button) to display an indication related to the determined concentration of the lactate. In this example, the indication comprises a numerical value for the determined concentration of lactate, and is provided on a second screenshot. On the second screen, the user is provided with a prompt to either return to the first screen or to proceed to a third screen. If the user selects to proceed to a third screen, they are presented with indications related to the determined concentrations of glucose, lactate and ketone. In this example, the user is presented with a numerical indication of the determined glucose concentration, a numerical indication of the determined lactate concentration, and a numerical indication of the determined ketone concentration. In this example, the determined concentration of ketones in the fluid sample is substantially zero.

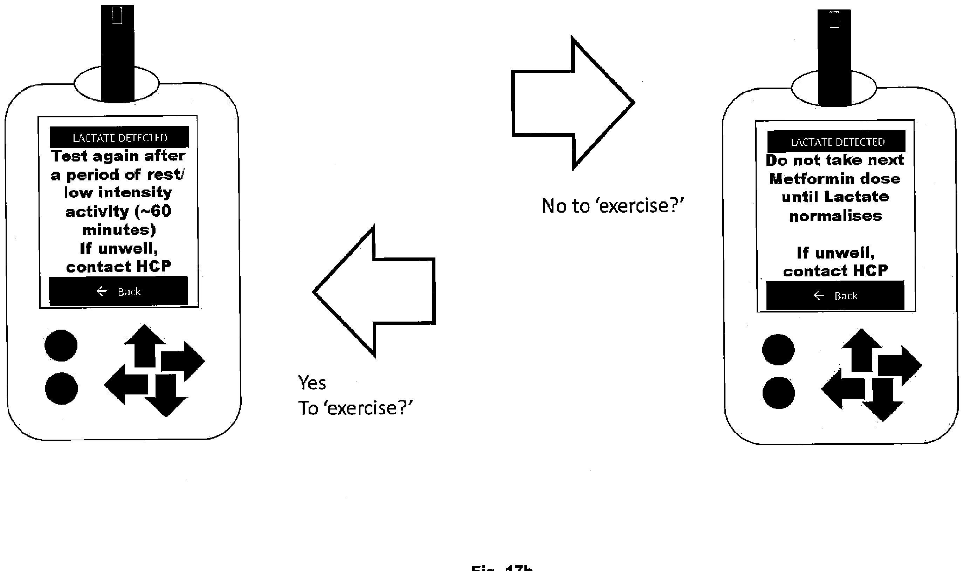

As the lactate level is above a threshold, the user is asked to input whether or not they have recently been exercising hard, as this may be an indication that a high lactate measurement is due to anaerobic exercise (see discussion above in relation to Figure 14 for further details). If the user indicates that they have been exercising hard, then they are prompted (see left hand screenshot of Figure 17b) to rest or perform low intensity activity for a period and then to perform another test to determine the concentration of lactate in their body. The user is further recommended to contact a health care professional if feeling unwell. If the user instead indicates that they have not been exercising hard then the user is recommended (see right hand screenshot of Figure 17b) to not take the next dose of Metformin due, and to contact a health care professional if feeling unwell.

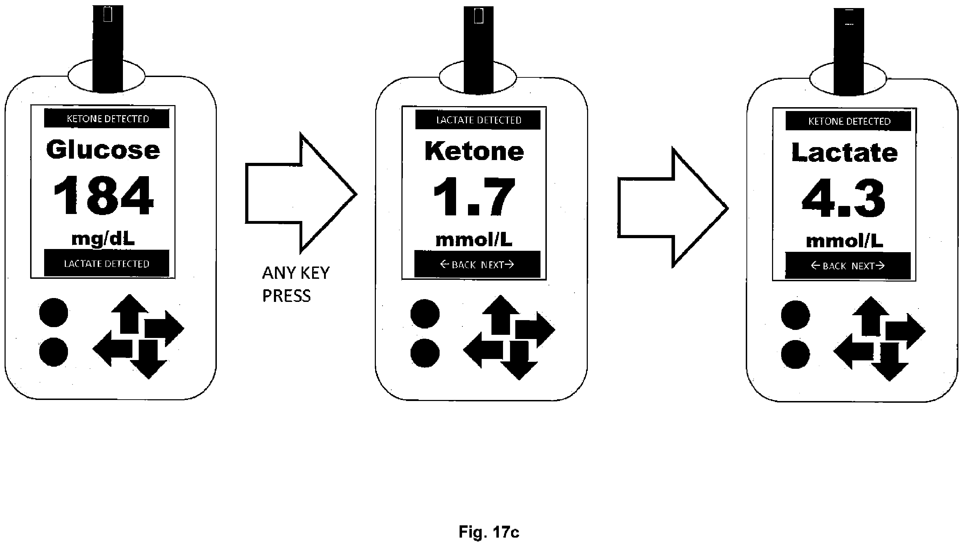

Figure 17c shows example screenshots of an apparatus or test meter when different concentrations of glucose, lactate and ketones are determined to those discussed above in relation to Figures 17a and 17b. In this example, the determined concentration of glucose is 184 mg/dL, the determined concentration of ketones is 1.7 mmol/L and the determined concentration of lactate is 4.3 mmol/L. The user is provided, on a first screen, with an indication of the determined concentration of glucose in the fluid sample. In this example, the indication comprises a numerical value of the determined glucose concentration. As the determined ketone value is above a threshold, a user-selectable option is provided to display, on a second screen, an indication related to the determined concentration of the ketone. In this example, the indication comprises a numerical value for the determined ketone concentration. A further user-selectable option to display, on a third screen, an indication related to a determined concentration of lactate is also provided. In this example, the indication comprises a numerical value for the determined lactate concentration.

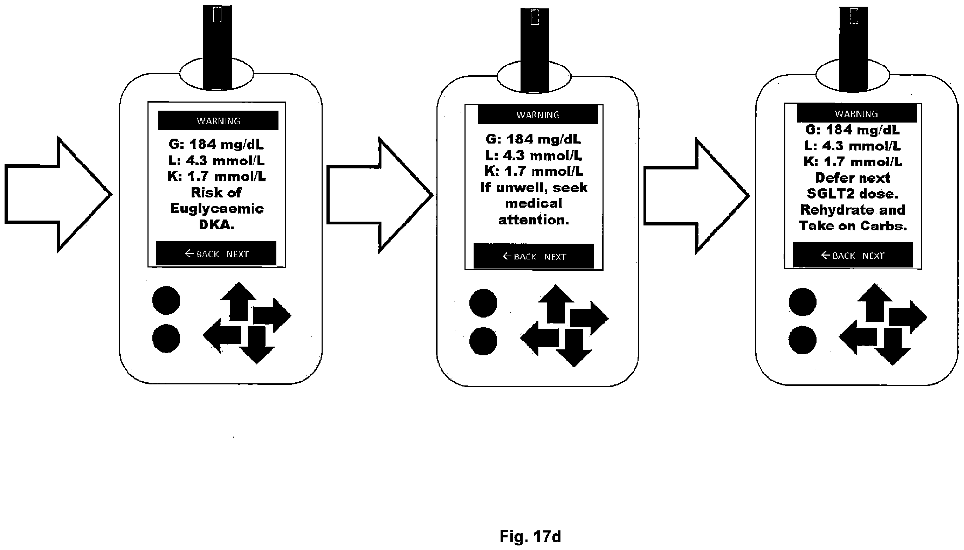

A user-selectable option to return to the previous screen is provided. A further option to proceed to a fourth screen (Figure 17d) is provided. As the determined glucose concentration is within a normal, euglycaemic, range and the determined lactate concentration is also not above a threshold, the user is presented with a warning on the fourth screen that they are at risk of euglycaemic DKA. A user-selectable option to return to the previous screen is provided. A further option to proceed to a fifth screen is provided. On the fifth screen, a recommendation to the user is provided. In this example, the recommendation is to seek medical attention if feeling unwell. A user-selectable option to return to the previous screen is provided. A further option to proceed to a sixth screen is provided. On the sixth screen, further recommendations are provided to the user. In this example, the user is recommended to defer taking of the next dose of medication, to rehydrate, and to consume carbohydrates.

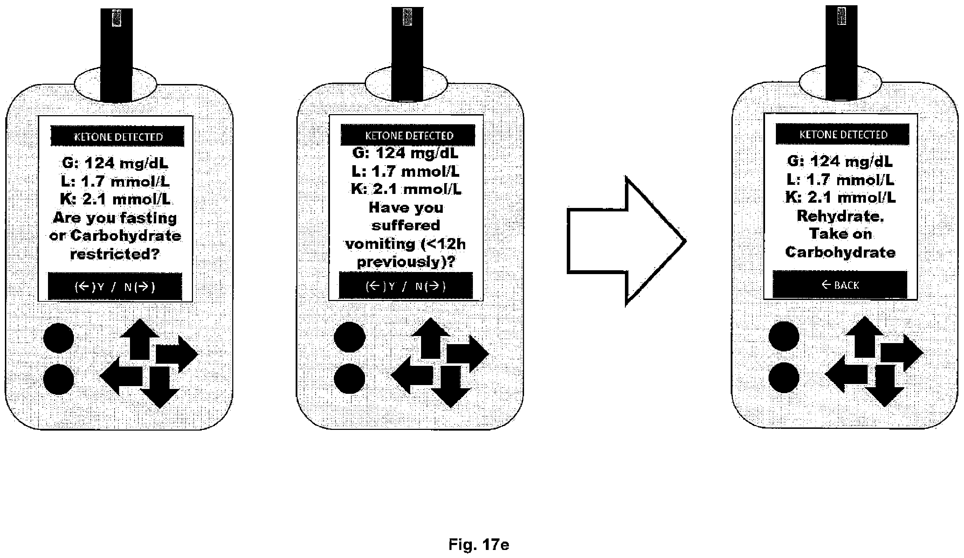

Figure 17e shows example screenshots of an apparatus or test meter when different concentrations of glucose, lactate and ketones are determined to those discussed above in relation to Figures 17a and 17b, or Figures 17c and 17d. In this example, the determined concentration of glucose is 124 mg/dL, the determined concentration of lactate is 1.7 mmol/L and the determined concentration of ketone is 2.1 mmol/L. The user is prompted to input information concerning whether or not they are fasting, and whether or not they have suffered vomiting in the twelve hours preceding the test. A recommendation is made that the user rehydrate and consume carbohydrates.

Variations of the described embodiments are envisaged, for example, the features of all the disclosed embodiments may be combined in any way.

For example, an electrochemical test device may contain more layers than those disclosed in the preceding description. For example, an electrochemical test device may further comprise one or more bonding layers for bonding together one or more of the layers disclosed above. Additionally, some of the layers are not always necessary. For example, the insulator layer may be absent from the examples discussed above. The spacer layer may define the interaction area of the electrodes of the conductor layer beneath. The spacer layer may perform the dual role of receiving a fluid sample through a capillary channel and defining an interaction area for combining the fluid sample with the conductor layer. For example, the spacer layer can, with appropriate adhesive, define the active area/interaction area of the electrodes.

In the examples of the electrochemical test device discussed above, a layer structure has been shown. The order in which each of the layers is formed may vary and any layer may, in some way, be configured so as to be in contact with any other layer.

The present disclosure is equally applicable to a glucose, ketone, lactate ("GKL") test strip. That is, in examples, there are provided three electrodes, one for each analyte.

The fluid sample may be a biological fluid. For example, the biological fluid may be blood, interstitial fluid, plasma, sweat, urine, lachrymal fluid, saliva or breath condensate. The one or more analytes may be any analyte(s) found in the fluid sample. For example, the analytes may be glucose and one or more of lactate, a ketone body such as β-hydroxybutyrate, bicarbonate, pH, or a kidney function analyte. Other ketone bodies that may be detected include acetoactetate, acetone, β-hydroxy pentanoate, and β-keto pentanoate.

The electrochemical test device may be configured to detect any combination of analytes so long as a suitable sensing chemistry is used. Example combinations include glucose and β-hydroxybutyrate; glucose and lactate; and lactate and β-hydroxybutyrate to name a few. Further working electrodes may be provided allowing higher numbers of analytes to be measured. For example, the electrochemical test strip may be configured to detect glucose, lactate and β-hydroxybutyrate.

The electrochemical test device may be any suitable electrochemical test device. The electrochemical test device may be a test strip. In some examples the electrochemical test device may comprise a patch. Electrochemical test devices such as patches typically comprise a subcutaneous fluid extraction set and sensing chemistry for interaction with the one or more analytes. The electrochemical test device may be a monitoring component which transmits an output signal to a separate device such as a meter, either wirelessly or through a wired connection. The electrochemical test device may comprise a continuous monitoring device or a semi-continuous monitoring device.

In the examples discussed above in relation to Figure 1 and Figure 15, the electrochemical test devices had an end-fill configuration. In other examples, an electrochemical test device has a side-fill configuration i.e. the fluid sample is received at the side of the electrochemical test device.

The electrochemical test device may be suitable for measuring any fluid sample volume and may be of a suitable corresponding size for the volume. For example the electrochemical test devices described in relation to Figure 1 and Figure 15 were arranged to receive approximately 0.5 μl of blood. The electrochemical test device may be scaled so as to receive other volumes including, for example, between 0.5μl and 5μl of a fluid, or between 0.5μl and 1 μl of a fluid. The electrochemical test device may be scaled so as to receive less than 0.5μl of a fluid, for example around 0.2μl or around 0.3μl.

Although in the discussion above in relation to Figures 1 and 15 a fill-sufficiency detect electrode was present, the fill-sufficiency detect electrode need not be present. Additionally, the fill-sufficiency detect electrode may or may not be coated in one or more reagent layers.

In examples, the device is a layered device. In examples, the first working electrode, second working electrode and, optionally, third working electrode are disposed on one layer of the test device.

In the examples provided above, the conductor layer and the insulator layer are printed layers. The conductor layer and the insulator layer may be supplied using any suitable manufacturing technique. These include forms of printing, for example, screen printing, lithographic printing or tomographic printing. The conductor layer and the insulator layer need not be provided in the same way. Other suitable manufacturing techniques include etching, and/or sputtering, chemical vapour deposition or physical vapour deposition.

A conductor layer may be formed of any suitable conductor. For example, the conductor layer may be formed from a carbon based paste, such as a carbon/graphite paste, including graphene. The conductor layer may be formed of one or more metal based paste such as a gold, platinum or silver paste. Although the conductor layer 112 described above in relation to Figure 1, and the conductor layer 1512 described above in relation to Figure 15 comprise carbon-based ink, the conductors need not be formed from carbon based ink. For example, the electrodes may be formed of silver (Ag) or silver/silver chloride (Ag/AgCl). In some examples, the electrodes are formed of different conducting materials. The one or more working electrodes may, for example, be formed of carbon based ink whereas the counter/reference electrode may be formed of silver (Ag) or silver/silver chloride (Ag/AgCl).

The conductor layer may be of any suitable thickness. For example, the conductor layer may have a thickness greater than or equal to 0.005mm and less than or equal to 0.030mm.

The ordering of the electrodes on the electrochemical test device may be altered for efficiency. In one preferable option, an electrode for an analyte for which a weak signal is expected (for example β-hydroxybutyrate which is often present in very low concentrations in blood) may be positioned closer to the entrance of the sample introduction chamber than an electrode for an analyte such as glucose or lactate which is usually present in higher concentrations.

The insulator layer may be formed of any suitable insulating material. For example, dielectric/insulation inks may be polymer loaded inks that are thermoplastic, thermoset or UV cured and that, when dried or cured, form a contiguous non-conductive layer. Examples include, Loctite EDAG PF 021 E&C and DuPont 5018.

In the examples discussed above, a polyester substrate layer was featured. Suitable substrate materials include polyester, polyimide, polystyrene, PVC, polycarbonate, glass and ceramic. When other layers are to be printed onto the substrate layer, the substrate layer has to be suitably printable for the chosen inks. The substrate must also be non-conductive. Typical thicknesses of the substrate layer range from 0.1mm to 0.5mm e.g. 0.35mm. Glass and ceramic can be thicker as these are easier to handle with increased thickness. Thinner polymer substrates may be more difficult for the end user to use. Thicker substrates may offer some handling benefits.

The spacer layer may be formed of any suitable material. For example, the spacer layer may be made from a polyester core with a thin layer of PSA (Pressure Sensitive Adhesive) on either side. These adhesives can be the same or different depending on which layer is to be adhered to which side of the spacer layer.

Although in the examples above the thickness of the spacer layer was 0.1mm, the thickness may vary. A typical range for the spacer layer thickness is 0.005 - 0.030mm. Lower thicknesses may affect sensor performance and higher thicknesses would increase the volume of the sample introduction channel. A thickness of an adhesive on the spacer layer may contribute to the rigidity of the spacer layer.

Typically a spacer layer has a high volume resistivity. For example the volume resistivity may be greater than $1 x 10^9 \Omega cm$.

Other variations of the spacer layer are envisaged.

The sample introduction chamber may be provided along the longitudinal axis of the electrochemical device. The sample introduction chamber may be provided along the transverse axis of the electrochemical test device.

The vent may be of any suitable configuration for venting air from the sample introduction chamber. For example, the vent may comprise an air passageway in the cover. The vent may comprise an air passageway in the spacer layer. Optionally, air may be vented from the sample introduction chamber through one or more air passageways below the spacer layer, such as through the conductor layer or the insulator layer.

In the examples above, the sensing chemistry is applied to each of the working electrodes as two reagent layers. There may be more than two reagent layers. For example, there may be a number of intermediate layers between a layer adjacent the electrode and a layer for coming into contact with the fluid. There may only be one reagent layer on a working electrode.

Many electron transfer agents or mediators may be used with electrochemical test devices such as those described herein. For example, suitable mediators include quinones such as benzoquinone, dyes such as 2,6-dichlorophenolindophenol and tetrazolium dyes, and redox couples such as ferricyanide anions and ferricinium cations, phenazine ethosulfate, phenazine methosulfate, 2-methyl-1,4-naphthoquinone and ferrocene derivatives.

The electron transfer agent may comprise a suitable quinone, for example a naphthoquinone derivative. The naphthoquinone derivative may be a 1,2 naphthoquinone derivative or a 1,4 naphthoquinone derivative. For example, the electron transfer agent may comprise 1,4 naphthoquinone-2-mercapto methyl carboxylic acid which has a standard redox potential of around -0.355V. The electron transfer agent may comprise 1,4 naphthoquinone-2-mercapto benzoic acid, which has a standard redox potential of around -0.345V. The electron transfer agent may comprise 1,2 naphthoquinone-4-sulphonate, which has a standard redox potential of around -0.214V. The electron transfer agent may comprise 1,4 naphthoquinone-2-mercapto methyl sulphonate. Also other suitable isomers of the above listed compounds are known which have similarly low standard redox potentials.

Both ruthenium hexaammine trichloride and ruthenium pentaammine chloride were mentioned above as suitable mediators. Other ruthenium-based complexes that may be used as a mediator include ruthenium pentaammine 4-methyl pyridine and ruthenium pentaammine pyridine.

The electron transfer agent may be a ruthenium- or osmium-based electron transfer agent. The ruthenium- or osmium-based electron transfer agent may be a complex of formula (3),

$$[M(A)_x(B)_y](X)_n \qquad (3)$$

wherein M is ruthenium or osmium, A is an amine ligand, each B is a ligand different to A, x is an integer selected from 1 to 5, y is an integer selected from 1 to 5, x+y is 6 or 8, n is an integer selected from 1 to 6, and X is any suitable counterion.

M may be ruthenium. For example, M may be Ru(II) or Ru(III). The oxidation state of the metal M in the complex may be selected to be 2+, 3+ or 4+.

A may be NRR'R", wherein R, R' and R" are independently selected from hydrogen or alkyl. A may be $NH_3$. It will be appreciated that when x is two or more, all "A" may be the same.

Each B is a ligand different to A. It will be appreciated that when y is 2 or more, B may be the same or different. B may be independently selected from a halide or optionally substituted heteroaryl. When B is an optionally substituted heteroaryl, the heteroaryl may be optionally substituted with an optionally substituted $C_{1-6}$ alkyl. B may be a halide, and the halide may be selected from the group consisting of F⁻, Cl⁻, Br⁻, I⁻. B may be chloride. B may be pyridyl, or 4-methyl pyridyl.

It will be appreciated that A and B may be selected such that the overall charge on the complex of formula (1) is selected from the group +2, +1, 0, -1, -2 and -3.

The counterion X may be a counterion selected to lead to the charge neutrality of $[M(A)_x(B)_y]$. The counterion X may be selected from F⁻, Cl⁻, Br⁻, I⁻, $PF_6^-$.

The ruthenium complex may be [Ruthenium$^{III}$(NH$_3$)$_5$(pyridine)]X. The ruthenium complex may be [Ruthenium$^{III}$(NH$_3$)$_5$(4-methyl pyridine)]X. The ruthenium complex may be [Ruthenium$^{III}$(NH$_3$)$_5$Cl]X (ruthenium pentaammine chloride). The ruthenium complex may be [Ruthenium$^{III}$(NH$_3$)$_5$Cl].2Cl.

The counterion X may be a counterion selected to lead to the charge neutrality of $[M(A)_x(B)_y]$. The counterion X may be selected from F⁻, Cl⁻, Br⁻, I⁻, $PF_6^-$.

The ruthenium- or osmium-based electron agent may be a ruthenium-based electron transfer agent. The concentration of the ruthenium-based electron transfer agent in the sensing chemistry may be from 8% to 15% by weight before drying of the sensing chemistry.

Transition metal complexes of the present disclosure can be soluble in water or other aqueous solutions. In general, the transition metal complexes can be made soluble in aqueous solvents by having an appropriate counterion or ions, X. The solubility of the transition metal complex may be greater than about 0.025M at 25°C in water.

Suitable electron transfer agents may be osmium-based compounds. For example, osmium phendione may be used as a mediator. [Os(4,4'-dimethyl-2,2'-bipyridine)$_2$] may also be used as a mediator, as may [(trpy)(bpy)M-OH]$^{2+}$ (M=Os; trpy=2,2',2"-terpyridine; bpy-2,2'-bipyridine).

The sensing chemistry may comprise between about 0.3%-2% (w/w) diaphorase. The sensing chemistry may comprise about 1% (w/w) diaphorase.

The diaphorase may be dissolved in a buffer such as, for example, phosphate or citrate. The buffer may be Tris buffer. The pH of the buffer may be about 7.

The sensing chemistry may comprise a phosphate or Tris buffer. The pH of the buffer may be in the range of about 6.5 - 7.5. For example, the pH of the buffer may be about 7. The pH of the buffer may be in the range of about 9.5-11. For example, the pH of the buffer may be about 10.5. The buffer may be of any suitable pH.

The diaphorase may have an enzyme activity range of from about 75 kU to 200 kU per 100 grams composition. The enzyme activity range is selected so that the analyte current does not depend on the level of enzyme activity in the composition and to avoid solubility issues for too high levels of diaphorase.

The sensing chemistry may comprise between about 0.07% - 0.13% (w/w) flavin mononucleotide (FMN). The sensing chemistry may comprise 0.1% (w/w) FMN.

The sensing chemistry may comprise about 0.5% - 3.5% (w/w), or 2.5% - 3.5% (w/w), hydroxyethyl cellulose (HEC). The sensing chemistry may comprise 3% HEC.

In the context used herein, "about" may refer to a variation of $\pm$ 10% of the numerical value.

A reagent for reacting with an analyte in a fluid sample need not be an oxidase. For example, for detecting glucose, rather than a glucose oxidase the sensing chemistry of a working electrode may comprise a dehydrogenase, such as flavin adenine dinucleotide (FAD)-dependent glucose dehydrogenase with its FAD cofactor, or pyrroloquinoline quinone (PQQ)-dependent glucose dehydrogenase with its PQQ cofactor. Alternatively, a NAD(P)$^+$-dependent dehydrogenase such as NAD(P)$^+$-glucose dehydrogenase may be used. For example, for detecting lactate, rather than a lactate oxidase the sensing chemistry of a working electrode may comprise a dehydrogenase, such as a lactate dehydrogenase.

Whilst the above examples have been described primarily in the context of an electrochemical test device for measuring a concentration of an analyte in a bodily fluid, it may equally be used in other fields, for example in health and fitness, food, drink, bio-security applications and environmental sample monitoring. The examples described herein may equally be used in the context of animal/veterinary medicine and fitness (including dogs and horses).

The apparatus may be configured to receive one or more test devices in order to receive information for determining concentrations of measured analytes. Information received may be time-stamped so as to check for sample relevance. Information may be received from other analytical equipment.

The above embodiments have been described by way of example only, and the described embodiments are to be considered in all respects only as illustrative and not restrictive. It will be appreciated that variations of the described embodiments may be made without departing from the scope of the invention.

**Claims**

1. A method of processing data from a multi-analyte electrochemical test device, the method comprising:

   determining a concentration of a first analyte in a fluid sample using first sensing chemistry, a first working electrode, and a counter electrode, wherein the first analyte is an acidosis-related analyte and comprises a ketone body;
   determining a concentration of a second analyte in the fluid sample using second sensing chemistry, a second working electrode, and the counter electrode, wherein the second analyte is glucose;
   displaying a first indication related to the determined concentration of the second analyte;
   if the determined concentration of the first analyte is equal to or greater than a first threshold, wherein the first threshold is a user definable threshold, providing a user-selectable option to display a second indication related to the determined concentration of the first analyte.

2. A method according to any preceding claim, wherein the step of determining a concentration of a second analyte in the fluid sample comprises determining that the concentration of the second analyte is indicative of euglycaemia.

3. A method according to any preceding claim, wherein the step of determining a concentration of a second analyte in the fluid sample comprises determining that the concentration of the second analyte is less than or equal to 33 mmol/L and greater than or equal to 4 mmol/L.

4. A method according to claim 3, wherein the step of determining a concentration of a second analyte in the fluid sample comprises determining that the concentration of the second analyte is less than or equal to 11 mmol/L and greater than or equal to 4 mmol/L.

5. A method according to claim 1, wherein the step of determining a concentration of a second analyte in the fluid sample comprises determining that the concentration of the second analyte is indicative of hypoglycaemia.

6. A method according to claim 1, wherein the step of determining a concentration of a second analyte in the fluid sample comprises determining that the concentration of the second analyte is indicative of hyperglycaemia.

7. A method according to any preceding claim, further comprising receiving user selection of the user-selectable option and, in response to receiving user selection of the user-selectable option, stopping display of the first indication and displaying the second indication.

8. A method according to any preceding claim, wherein determining a concentration of a first analyte in the fluid sample comprises determining that the concentration of the first analyte is indicative of an acidosis-related condition.

9. A method according to claim 8, wherein the acidosis-related condition is any one of:

   diabetic ketoacidosis, DKA; or
   euglycaemic diabetic ketoacidosis; or
   Metformin associated lactic acidosis, MALA.

10. A method according to any preceding claim, wherein the first analyte comprises β-hydroxybutyrate.

11. A method according to any preceding claim, wherein the first threshold is about 0.1 mmol/L, optionally wherein:

    if the determined concentration of the first analyte is greater than or equal to a second threshold, the second threshold being either:

    0.6 mmol/L; or
    1.5 mmol/L;

    the second indication comprises a warning concerning the determined concentration of the first analyte.

12. A method according to any preceding claim, wherein the first threshold is about 0.6 mmol/L. 28, optionally wherein:

    if the determined concentration of the first analyte is greater than or equal to a second threshold, the second threshold being either:

    1.5 mmol/L; or
    3 mmol/L ;

    the second indication comprises a warning concerning the determined concentration of the first analyte.

13. A method according to any preceding claim, wherein the first threshold is about 1.5 mmol/L. 30, optionally wherein:

    if the determined concentration of the first analyte is greater than or equal to a second threshold, the second threshold being either:

    3 mmol/L; or
    5 mmol/L;

    the second indication comprises a warning concerning the determined concentration of the first analyte.

14. A method according to any of claims 1 - 10, wherein if the determined concentration of the first analyte is greater than or equal to a second threshold, the second threshold being greater than the first threshold, the second indication comprises a warning concerning the determined concentration of the first analyte.

15. A method according to claim 14, wherein the second threshold is a user definable threshold.

**Patentansprüche**

1. Verfahren zum Verarbeiten von Daten von einer elektrochemischen Mehranalyt-Testvorrichtung, das Verfahren umfassend:

   Bestimmen einer Konzentration eines ersten Analyts in einer Fluidprobe unter Verwendung erster Erfassungschemie, einer ersten Arbeitselektrode und einer Gegenelektrode, wobei der erste Analyt ein zu Azidose in Beziehung stehender Analyt ist und einen Ketonkörper umfasst;
   Bestimmen einer Konzentration eines zweiten Analyts in der Fluidprobe unter Verwendung zweiter Erfassungschemie, einer zweiten Arbeitselektrode und der Gegenelektrode, wobei der zweite Analyt Glucose ist;
   Anzeigen einer ersten Angabe hinsichtlich der bestimmten Konzentration des zweiten Analyts;
   wenn die bestimmte Konzentration des ersten Analyts gleich dem oder größer als ein erster Schwellenwert ist, wobei der erste Schwellenwert ein durch den Benutzer definierbarer Schwellenwert ist, Bereitstellen einer durch den Benutzer auswählbaren Option zum Anzeigen einer zweiten Angabe hinsichtlich der bestimmten Konzentration des ersten Analyts.

2. Verfahren nach einem der vorstehenden Ansprüche, wobei der Schritt des Bestimmens einer Konzentration eines zweiten Analyts in der Fluidprobe umfasst, bestimmen, dass die Konzentration des zweiten Analyts Euglykämie angibt.

3. Verfahren nach einem der vorstehenden Ansprüche, wobei der Schritt des Bestimmens einer Konzentration eines zweiten Analyts in der Fluidprobe umfasst, bestimmen, dass die Konzentration des zweiten Analyts kleiner als oder gleich 33 mmol/L und größer als oder gleich 4 mmol/L ist.

4. Verfahren nach Anspruch 3, wobei der Schritt des Bestimmens einer Konzentration eines zweiten Analyts in der Fluidprobe umfasst, bestimmen, dass die Konzentration des zweiten Analyts kleiner als oder gleich 11 mmol/L und größer als oder gleich 4 mmol/L ist.

5. Verfahren nach Anspruch 1, wobei der Schritt des Bestimmens einer Konzentration eines zweiten Analyts in der Fluidprobe umfasst, bestimmen, dass die Konzentration des zweiten Analyts Hypoglykämie angibt.

6. Verfahren nach Anspruch 1, wobei der Schritt des Bestimmens einer Konzentration eines zweiten Analyts in der Fluidprobe umfasst, bestimmen, dass die Konzentration des zweiten Analyts Hyperglykämie angibt.

7. Verfahren nach einem der vorstehenden Ansprüche, ferner umfassend Empfangen einer Benutzerauswahl der durch den Benutzer auswählbaren Option und, als Reaktion auf Empfangen der Benutzerauswahl der durch den Benutzer auswählbaren Option, Stoppen der Anzeige der ersten Angabe und Anzeigen der zweiten Angabe.

8. Verfahren nach einem der vorstehenden Ansprüche, wobei Bestimmen einer Konzentration eines ersten Analyts in der Fluidprobe umfasst, bestimmen, dass die Konzentration des ersten Analyts einen zu Azidose in Beziehung stehenden Zustand angibt.

9. Verfahren nach Anspruch 8, wobei der zu Azidose in Beziehung stehende Zustand einer von folgenden ist:

   diabetische Ketoazidose, DKA; oder
   euglykämische diabetische Ketoazidose; oder
   Metformin-assoziierte Milchsäureazidose, MALA.

10. Verfahren nach einem der vorstehenden Ansprüche, wobei der erste Analyt β-Hydroxybutyrat umfasst.

11. Verfahren nach einem der vorstehenden Ansprüche, wobei der erste Schwellenwert etwa 0,1 mmol/L beträgt, wahlweise wobei:

   wenn die bestimmte Konzentration des ersten Analyts größer als oder gleich einem zweiten Schwellenwert ist, der zweite Schwellenwert entweder:

      0,6 mmol/L; oder
      1,5 mmol/L beträgt;

   wobei die zweite Angabe eine Warnung bezüglich der bestimmten Konzentration des ersten Analyts umfasst.

12. Verfahren nach einem der vorstehenden Ansprüche, wobei der erste Schwellenwert etwa 0,6 mmol/L. 28 beträgt, wahlweise wobei:

   wenn die bestimmte Konzentration des ersten Analyts größer als oder gleich einem zweiten Schwellenwert ist, der zweite Schwellenwert entweder:

      1,5 mmol/L; oder
      3 mmol/L beträgt;

   wobei die zweite Angabe eine Warnung bezüglich der bestimmten Konzentration des ersten Analyts umfasst.

**13.** Verfahren nach einem der vorstehenden Ansprüche, wobei der erste Schwellenwert etwa 1,5 mmol/L. 30 beträgt, wahlweise wobei:

wenn die bestimmte Konzentration des ersten Analyts größer als oder gleich einem zweiten Schwellenwert ist, der zweite Schwellenwert entweder:

3 mmol/L; oder
5 mmol/L beträgt;

wobei die zweite Angabe eine Warnung bezüglich der bestimmten Konzentration des ersten Analyts umfasst.

**14.** Verfahren nach einem der Ansprüche 1-10, wobei, wenn die bestimmte Konzentration des ersten Analyts größer als oder gleich einem zweiten Schwellenwert ist, wobei der zweite Schwellenwert größer als der erste Schwellenwert ist, die zweite Angabe eine Warnung bezüglich der bestimmten Konzentration des ersten Analyts umfasst.

**15.** Verfahren nach Anspruch 14, wobei der zweite Schwellenwert ein durch den Benutzer definierbarer Schwellenwert ist.


**Revendications**

**1.** Procédé pour le traitement de données provenant d'un dispositif de test électrochimique à analytes multiples, le procédé comprenant:

la détermination d'une concentration d'un premier analyte dans un échantillon de fluide en utilisant une première chimie de détection, une première électrode de travail et une contre-électrode, dans lequel le premier analyte est un analyte lié à une acidose et comprend un corps cétonique;
la détermination d'une concentration d'un deuxième analyte dans l'échantillon de fluide en utilisant une deuxième chimie de détection, une deuxième électrode de travail et la contre-électrode, dans lequel le deuxième analyte est du glucose;
l'affichage d'une première indication en rapport avec la concentration déterminée du deuxième analyte;
si la concentration déterminée du premier analyte est supérieure ou égale à un premier seuil, où le premier seuil est un seuil définissable par l'utilisateur, la fourniture d'une option sélectionnable par l'utilisateur d'afficher une deuxième indication en rapport avec la concentration déterminée du premier analyte.

**2.** Procédé selon l'une quelconque des revendications précédentes, dans lequel l'étape de détermination d'une concentration d'un deuxième analyte dans l'échantillon de fluide comprend la détermination que la concentration du deuxième analyte est une indication d'une euglycémie.

**3.** Procédé selon l'une quelconque des revendications précédentes, dans lequel l'étape de détermination d'une concentration d'un deuxième analyte dans l'échantillon de fluide comprend la détermination que la concentration du deuxième analyte est inférieure ou égale à 33 mmol/l et supérieure ou égale à 4 mmol/l/

**4.** Procédé selon la revendication 3, dans lequel l'étape de détermination d'une concentration d'un deuxième analyte dans l'échantillon de fluide comprend la détermination que la concentration du deuxième analyte est inférieure ou égale à 11 mmol/l et supérieure ou égale à 4 mmol/l.

**5.** Procédé selon la revendication 1, dans lequel l'étape de détermination d'une concentration d'un deuxième analyte dans l'échantillon de fluide comprend la détermination que la concentration du deuxième analyte est une indication d'une hypoglycémie.

**6.** Procédé selon la revendication 1, dans lequel l'étape de détermination d'une concentration d'un deuxième analyte dans l'échantillon de fluide comprend la détermination que la concentration du deuxième analyte est une indication d'une hyperglycémie.

**7.** Procédé selon l'une quelconque des revendications précédentes, comprenant en outre la réception d'une sélection de l'utilisateur de l'option sélectionnable par l'utilisateur et, en réponse à la réception de la sélection de l'utilisateur de l'option sélectionnable par l'utilisateur, l'arrêt de l'affichage de la première indication et l'affichage de la deuxième indication.

8. Procédé selon l'une quelconque des revendications précédentes, dans lequel la détermination d'une concentration d'un premier analyte dans l'échantillon de fluide comprend la détermination que la concentration du premier analyte est une indication d'un état lié à une acidose.

9. Procédé selon la revendication 8, dans lequel l'état lié à une acidose est n'importe lequel parmi:

une cétoacidose diabétique, DKA; ou
une cétoacidose diabétique euglycémique; ou
une acidose lactique associée à la métformine, MALA.

10. Procédé selon l'une quelconque des revendications précédentes, dans lequel le premier analyte comprend du β-hydroxybutyrate.

11. Procédé selon l'une quelconque des revendications précédentes, dans lequel le premier seuil est environ 0,1 mmol/l, optionnellement dans lequel:

si la concentration déterminée du premier analyte est supérieure ou égale à un deuxième seuil, le deuxième seuil étant:

soit 0,6 mmol/l;
soit 1,5 mmol/l;

la deuxième indication comprend un avertissement concernant la concentration déterminée du premier analyte.

12. Procédé selon l'une quelconque des revendications précédentes, dans lequel le premier seuil est environ 0,6 mmol/l. 28, optionnellement dans lequel:

si la concentration déterminée du premier analyte est supérieure ou égale à un deuxième seuil, le deuxième seuil étant:

soit 1,5 mmol/l;
soit 3 mmol/l;

la deuxième indication comprend un avertissement concernant la concentration déterminée du premier analyte.

13. Procédé selon l'une quelconque des revendications précédentes, dans lequel le premier seuil est environ 1,5 mmol/l. 30, optionnellement dans lequel:

si la concentration déterminée du premier analyte est supérieure ou égale à un deuxième seuil, le deuxième seuil étant:

soit 3 mmol/l;
soit 5 mmol/l;

la deuxième indication comprend un avertissement concernant la concentration déterminée du premier analyte.

14. Procédé selon l'une quelconque des revendications 1-10, dans lequel si la concentration déterminée du premier analyte est supérieure ou égale à un deuxième seuil, le deuxième seuil étant supérieur au premier seuil, la deuxième indication comprend un avertissement concernant la concentration déterminée du premier analyte.

15. Procédé selon la revendication 14, dans lequel le deuxième seuil est un seuil définissable par l'utilisateur.

**Fig. 1**

Fig. 2

**Fig. 3**

| |
|---|
| **440** |
| **430** |
| **410** |

| |
|---|
| **460** |
| **450** |
| **420** |

Fig. 4

| |
|---|
| **540** |
| **530** |
| **410** |

| |
|---|
| **560** |
| **550** |
| **420** |

Fig. 5

**600**

| Processor | Memory | Power System |
|-----------|--------|--------------|
| 614 | 616 | 618 |

| Visual Display | User input device | Communications module | Port |
|----------------|-------------------|------------------------|------|
| 610 | 612 | 620 | 622 |

Fig. 6

710 — Determine a concentration of a first analyte in a fluid sample

720 — Determine a concentration of a second analyte in the fluid sample

730 — Display a first indication related to the determined concentration of the second analyte

740 — If the determined concentration of the first analyte is equal to or greater than a first threshold, provide a user-selectable option to display a second indication related to the determined concentration of the first analyte

**Fig. 7**

Fig. 8

**910** System set up by power user or HCP

**920** "sustained" EuDKA risk level determined

**930** Test performed

**940** Condition indicated

**950** Condition assessed

**960** Interpreted ketone result communicated to user with action and/or advice if appropriate

**970** Glucose result communicated immediately

**980** Ketone result displayed immediately if DKA risk is severe

**990** User prompted for additional input if appropriate

**995** Transient EuDKA risk level assessed

**Fig. 9**

| Glucose trend: | downward | |
| 2 wk. average- | 135 | now |
| 2 wk. average- | 150 | last week |
| 2 wk. average- | 175 | 2 weeks ago |
| Ketones | present/increasing | |
| over 2 weeks | 2/24>0.6mmol/L | now |
| over 2 weeks | absent | last week |
| over 2 weeks | absent | 2 weeks ago |

← back    more →

## Have you been taking your SGLT2 correctly?

## Is your dose too high?

Press → to view details

Fig. 10a

**Hyperglycaemia** — Decreasing
over 2 weeks — 1/24 >200 mg/dl — now
over 2 weeks — 3/24 >200 mg/dl — last week
over 2 weeks — 4/24 >200 mg/dk — 2 weeks ago

**Hypoglycaemia** — Stable
over 2 weeks — 0/24 <70 mg/dl — now
over 2 weeks — 0/24 <70 mg/dl — last week
over 2 weeks — 0/24 <70 mg/dk — 2 weeks ago

← back        more →

**Euglycaemia**
over 2 weeks — 22/24 — now
over 2 weeks — 21/24 — last week
over 2 weeks — 20/24 >200 mg/dk — 2 weeks ago

**mild acidosis risk**
over 2 weeks — 2/24 — now
over 2 weeks — 0/24 — last week
over 2 weeks — 0/24 — 2 weeks ago

← back        more →

Fig. 10b

| moderate DKA risk | | absent |
| over2 weeks | 0/24 | now |
| over2 weeks | 0/24 | last week |
| over2 weeks | 0/24 | 2 weeks ago |
| | | |
| severe DKA risk | | absent |
| over2 weeks | 0/24 | now |
| over2 weeks | 0/24 | last week |
| over2 weeks | 0/24 | 2 weeks ago |

← back   more →

Fig. 10c

Right arrow
pressed

Ketone above CT1

Fig. 11a

EP 3 310 925 B1

Fig. 11b

Suspect hyper – glycaemia. Take action to reduce blood sugar. Glucose 213 mg/dL

← BACK

UP arrow Pressed

Ketone above CT1

↑NEXT SCREEN
Glucose
213
mg/dL
VIEW KETONES →

**Fig. 11c**

Fig. 12

Fig. 13

Patient is taking Metformin or other biguanide based medication **1402**

Elevated lactate is detected **1404**

Instigate hiatus in Metformin therapy **1414**

Has patient been exercising strenuously? **1406**

Y → Probably exercise induced lactate build up from muscles. Retest after sedentary period **1408**

Retest indicates lactate is normalising? **1410** N

Y → Benign event **1412**

Hospitalisation avoided

N → Prompt for ketone testing (blood, urine). DKA present? (Elevated BG, ketonuria) Prompt for DKA symptoms. **1418**

Y → Advise and treat for DKA (insulin/rehydration) **1420**

Resume Metformin when safe to do so **1422**

N → Sepsis or ischemia indicated (response to user prompts)? **1424**

Y → Manage sepsis/ischemia related condition. And treat lactic acidosis **1426**

Resume Metformin when safe to do so **1428**

N → Suspect MALA. Symptoms of MALA present? **1430**

Y → Recommend haemodialysis in the ER **1432**

Check kidney function before resuming Metformin therapy if still appropriate **1436**

Lactate normalises upon Metformin cessation? **1434**

N

Potentially life saving treatment facilitated in a timely manner

Fig. 14

56

**Fig. 15**

Fig. 16

**Fig. 17a**

EP 3 310 925 B1

EP 3 310 925 B1

**LACTATE DETECTED**

**Test again after a period of rest/ low intensity activity (~60 minutes) If unwell, contact HCP**

← Back

No to 'exercise?'

Yes
To 'exercise?'

**LACTATE DETECTED**

**Do not take next Metformin dose until Lactate normalises**

**If unwell, contact HCP**

← Back

Fig. 17b

**Fig. 17c**

EP 3 310 925 B1

Fig. 17d

EP 3 310 925 B1

KETONE DETECTED

G: 124 mg/dL
L: 1.7 mmol/L
K: 2.1 mmol/L
Are you fasting
or Carbohydrate
restricted?

( ← )Y / N( → )

KETONE DETECTED

G: 124 mg/dL
L: 1.7 mmol/L
K: 2.1 mmol/L
Have you
suffered
vomiting (<12h
previously)?

( ← )Y / N( → )

KETONE DETECTED

G: 124 mg/dL
L: 1.7 mmol/L
K: 2.1 mmol/L
Rehydrate.
Take on
Carbohydrate

← BACK

Fig. 17e

**REFERENCES CITED IN THE DESCRIPTION**

**Patent documents cited in the description**

- EP 16720526
- WO 2016174457 A
- EP 16720528
- EP 16720525
- EP 16720529